# EUROPEAN PATENT APPLICATION

(11) **EP 4 194 545 A1**
(43) Date of publication of application: **14.06.2023**
(21) Application number: 21854280.1
(22) Date of filing: 04.01.2021
(51) Int. Cl.: C12N 1/21, C12N 15/31, C12N 15/77, C07K 14/34, C12P 13/14, C12P 13/08, C12R 1/15, C12R 1/13

(54) **RECOMBINANT STRAIN FOR PRODUCING L-AMINO ACID, AND CONSTRUCTION METHOD THEREFOR AND USE THEREOF**

(30) Priority: 07.08.2020 CN 202010790868; 13.10.2020 CN 202011093080; 15.10.2020 CN 202011105035
(71) Applicant: Ningxia Eppen Biotech Co. Ltd, Ningxia 750100 (CN)
(72) Inventor: MENG, Gang, Yinchuan, Ningxia 750100 (CN); ZHAO, Chunguang, Yinchuan, Ningxia 750100 (CN); WEI, Aiying, Yinchuan, Ningxia 750100 (CN); ZHOU, Xiaoqun, Yinchuan, Ningxia 750100 (CN); MA, Fengyong, Yinchuan, Ningxia 750100 (CN); YANG, Lipeng, Yinchuan, Ningxia 750100 (CN); SU, Houbo, Yinchuan, Ningxia 750100 (CN); JIA, Huiping, Yinchuan, Ningxia 750100 (CN); TIAN, Bin, Yinchuan, Ningxia 750100 (CN); GUO, Xiaowei, Yinchuan, Ningxia 750100 (CN)
(74) Representative: Isarpatent
(86) International application number: PCT/CN2021/070190
(87) International publication number: WO 2022/027924

(57) **Abstract**

Taking Corynebacterium glutamicum YP97158 as the starting bacterium, introducing site-directed mutation and/or expression enhancement in the coding region of its NCgl1089 gene, the coding region of NCgl0761 gene, and/or the coding region of ptsS gene, the obtained mutant gene and the recombination comprising said gene has high-efficiency L-amino acids production capacity, which greatly increases the output of L-amino acids, and the strain has good stability, which reduces the production cost as an L-amino acids production strain.

## Description

The present invention claims the priority of the prior application with the patent application number 2020107908681 filed with the China National Intellectual Property Administration on August 7, 2020, and the prior application with the patent application number 2020111050353 filed with the China National Intellectual Property Administration on October 15, 2020, and also the priority of the prior application with the patent application number 2020110930801 filed with the China National Intellectual Property Administration on October 13, 2020, the entire contents of which are incorporated herein by reference.

### FIELD OF THE INVENTION

The present invention belongs to the technical field of genetic engineering and microorganisms, and in particular relates to a Corynebacterium strain with enhanced L-amino acids production capacity and a construction method and use thereof.

### BACKGROUND OF THE INVENTION

The production of L-amino acids is carried out industrially by fermentation using microorganisms such as bacteria having L-amino acids production capacity. As such microorganisms, for example, strains isolated from nature and mutant strains thereof can be used.

Improvements to the production of L-amino acids by fermentation may involve fermentation techniques such as stirring and supplying oxygen; or the composition of the nutrient medium, such as the sugar concentration during fermentation; or the processing of the fermentation broth into a suitable product form, such as by drying and pelleted fermentation broth or ion exchange chromatography; or may involve inherent performance properties of the relevant microorganism itself. Methods for improving the performance properties of these microorganisms include mutagenesis, selection and screening of mutants. Strains obtained in this way are resistant to antimetabolites or are auxotrophic for metabolites of regulatory importance and produce L-amino acids. There is still a need to develop methods for more economical production of L-amino acids in high yields

### SUMMARY OF THE INVENTION

The present invention provides a recombinant strain for producing L-amino acids, recombinant construction method, and use in fermentation production of amino acid thereof.

The present invention takes Corynebacterium glutamicum YP97158 as the starting bacterium, and introduces site-directed mutation and/or improved expression in its coding region of the NCgl1089 gene, and/or coding region of the NCgl0761 gene, and/or coding region of the ptsS gene, and the obtained mutant gene and the recombinant strain comprising the gene have efficient L-amino acid production capacity, which greatly increases the production of L-amino acids, and the strain is stable, reducing production costs as an L-amino acid production strain.

Therefore, the present invention provides the following technical solutions:

The present invention provides a microorganism belonging to the Corynebacterium that generates L-amino acids, which has an improved expression of the polynucleotide coding the amino acid sequence of SEQ ID NO:3, and/or SEQ ID NO:35, and/or SEQ ID NO:63. According to the present invention, the improved expression is an enhanced expression of the polynucleotide, or the polynucleotide coding the amino acid sequence has a point mutation, or the polynucleotide coding the amino acid sequence has a point mutation and the expression is enhanced.

The first aspect of the present invention:

The amino acid sequence of SEQ ID NO: 3 is a protein coded by the gene NCgl1089.

The polynucleotide may code an amino acid sequence that is about 90% or more, about 92% or more, about 95% or more, about 97% or more, about 98% or more, about 99% or more sequence homology to the amino acid sequence of SEQ ID NO:3.

In one embodiment of the present invention, the polynucleotide may include the nucleotide sequence of SEQ ID NO:1.

In one embodiment of the present invention, the polynucleotide coding the amino acid sequence of SEQ ID NO:3 has a point mutation such that the glutamic acid at position 170 of the amino acid sequence of SEQ ID NO:3 is replaced by different amino acids.

According to the present invention, preferably the glutamic acid at position 170 is replaced by lysine.

According to the present invention, the amino acid sequence shown in SEQ ID NO:3, wherein the amino acid sequence after the 170^{th} glutamic acid (E) is replaced by lysine (K) is shown in SEQ ID NO:4.

In one embodiment of the present invention, the polynucleotide sequence with point mutation is formed by the mutation of the 508^{th} base of the polynucleotide sequence shown in SEQ ID NO: 1.

According to the present invention, the mutation includes the mutation of guanine (G) to adenine (A) at the 508^{th} base of the polynucleotide sequence shown in SEQ ID NO: 1.

In one embodiment of the present invention, the polynucleotide sequence with point mutation includes the polynucleotide sequence shown in SEQ ID NO:2.

According to the present invention, the expression regulatory sequences of the polynucleotides of the present invention can be modified. Expression regulatory sequences control the expression of the polynucleotide to which it is operably linked. The regulatory sequences can be, for example, promoters, terminators, enhancers, silencers and the like.

In one embodiment of the present invention, the promoter is the promoter of the polynucleotide coding the amino acid sequence of SEQ ID NO:3 (NCgl1089 gene).

The present invention further provides a polynucleotide sequence, an amino acid sequence coded by the polynucleotide sequence, a recombinant vector including the polynucleotide sequence, and a recombinant strain containing the polynucleotide sequence.

According to the present invention, the polynucleotide sequence includes a polynucleotide coding the amino acid sequence shown in SEQ ID NO: 3, and the 170^{th} glutamic acid of which is replaced by different amino acids.

According to the present invention, preferably the glutamic acid at position 170 is replaced by lysine.

According to the present invention, the amino acid sequence shown in SEQ ID NO:3, wherein the amino acid sequence after the 170^{th} glutamic acid (E) is replaced by lysine (K) is shown in SEQ ID NO:4.

According to the present invention, preferably the polynucleotide sequence coding the amino acid sequence shown in SEQ ID NO: 3 contains the polynucleotide sequence shown in SEQ ID NO:1.

In one embodiment of the present invention, the polynucleotide sequence is formed by the mutation of the 508^{th} base of the polynucleotide sequence shown in SEQ ID NO: 1.

According to the present invention, the mutation includes the mutation of guanine (G) to adenine (A) at the 508^{th} base of the polynucleotide sequence shown in SEQ ID NO: 1.

In one embodiment of the present invention, the polynucleotide sequence includes the polynucleotide sequence shown in SEQ ID NO:2.

According to the present invention, the amino acid sequence includes the amino acid sequence shown in SEQ ID NO:4.

According to the present invention, the recombinant vector is constructed by introducing the polynucleotide sequence into a plasmid.

In one embodiment of the present invention, the plasmid is the pK18mobsacB plasmid.

In another embodiment of the present invention, the plasmid is the pXMJ19 plasmid.

Specifically, the polynucleotide sequence and the plasmid can be constructed into a recombinant vector by a NEBuider recombination system.

According to the present invention, the recombinant strain contains the polynucleotide sequence.

As an embodiment of the present invention, the starting bacterium of the recombinant strain is YP97158.

The present invention further provides a construction method of a Corynebacterium recombinant strain.

According to the present invention, the construction method includes the following steps:
The polynucleotide sequence of the wild-type NCgl1089 shown in SEQ ID NO: 1 in the host strain is transformed, and the 508th base is mutated to obtain a Corynebacterium recombinant strain including the gene coding the mutant NCgl1089.

According to the construction method of the present invention, the transformation includes at least one of mutagenesis, PCR site-directed mutagenesis, and/or homologous recombination.

According to the construction method of the present invention, the mutation refers to the mutation of guanine (G) to adenine (A) at the 508^{th} base in SEQ ID NO: 1; specifically, the polynucleotide sequence including the gene coding the mutant NCgl1089 is shown in SEQ ID NO:2.

Further, the construction method includes the following steps:
(1) transforming the nucleotide sequence of the wild-type NCgl1089 gene as shown in SEQ ID NO: 1, so that the 508^{th} base is mutated to obtain a mutated NCgl1089 gene polynucleotide sequence;
(2) passing the mutated polynucleotide sequence and plasmid through the NEBuider recombination system to construct a recombinant vector;
(3) introducing the recombinant vector into a host strain to obtain the Corynebacterium recombinant strain including the gene coding the mutant NCgl1089.

According to the construction method of the present invention, the step (1) includes: constructing a point-mutated NCgl1089 gene: according to the genome sequence of Corynebacterium glutamicum, synthesizing two pairs of primers P1 and P2 and P3 and P4 for amplifying the NCgl 1089 gene fragment, introducing apoint mutation into the wild-type NCgl1089 gene SEQ ID NO: 1 by PCR site-directed mutagenesis to obtain the point-mutated NCgl1089 gene nucleotide sequence SEQ ID NO: 2, denoted as NCgl1089 ^{G508A}.

In one embodiment of the present invention, the genome of Corynebacterium glutamicum can be derived from ATCC13032 strain, and its genome sequence can be obtained from the NCBI website.

In one embodiment of the present invention, in the step (1), the primers are:

P1:5'CAGTGCCAAGCTTGCATGCCTGCAGGTCGACTCTAGCGCGTGGATCCACGCCAG
3'(SEQ ID NO:5)

P2:S'CAATGAGGGCTTTCGCCACCTCGCGGGC 3'(SEQ ID NO:6)
P3:5'GCCCGCGAGGTGGCGAAAGCCCTCATTG 3'(SEQ ID NO:7)

P4:5'CAGCTATGACCATGATTACGAATTCGAGCTCGGTACCCCATGCGTTGGCGATCTTC
3'(SEQ ID NO:8).

In one embodiment of the present invention, the PCR amplification is performed as follows: denaturation at 94°C for 30s, annealing at 52°C for 30s, and extension at 72°C for 40s (30 cycles).

In one embodiment of the present invention, the overlap PCR amplification is performed as follows: denaturation at 94°C for 30s, annealing at 52°C for 30s, and extension at 72°C for 90s (30 cycles).

According to the construction method of the present invention, the step (2) includes the construction of the recombinant plasmid, including: assembling the isolated and purified NCgl1089^{G508A} and pK18mobsacB plasmids through the NEBuider recombination system to obtain the recombinant plasmid pK18-NCgl1089^{G508A}.

According to the construction method of the present invention, the step (3) includes the construction of a recombinant strain, and the recombinant plasmid pK18-NCgl1089 ^{G508A} is transformed into a host strain to obtain a recombinant strain.

In one embodiment of the present invention, the transformation of step (3) is an electrotransformation method.

In one embodiment of the present invention, the host strain is YP97158.

In one embodiment of the present invention, the recombination is implemented by homologous recombination.

The fourth aspect of the present invention further provides a construction method of a Corynebacterium recombinant strain.

According to the present invention, the construction method includes the following steps:
amplifying the upstream and downstream homology arm fragments of the NCgl1089 gene, the coding region of the NCgl1089 gene, the coding region of the NCgl1089^{G508A} gene, and the promoter sequence of the gene, and introducing NCgl1089 or NCgl1089 ^{G508A} gene into the genome of the host strain by homologous recombination to implement the strain overexpressing the NCgl1089 or NCgl1089^{G508A} gene.

In one embodiment of the present invention, the primers for amplifying the upstream homology arm fragment are:
P7:5'CAGTGCCAAGCTTGCATGCCTGCAGGTCGACTCTAGAATGCGTTCTGGACTGAGG 3'(SEQ ID NO:11)
P8: 5'CATGAGTATA AAATCACTGT CGTGCACCGAG AACAGATG 3'(SEQ ID NO:12).

In one embodiment of the present invention, the primers for amplifying the downstream homology arm fragments are:
P13: 5'CGTGCCCACAGAAGAGGTGAGAT GGCGCAATTA AATCAAG 3'(SEQ ID NO:17)

P14:5'CAGCTATGACCATGATTACGAATTCGAGCTCGGTACCCGCTATGACACCTTCAACGGA
TC 3'(SEQ ID NO:18)

In one embodiment of the present invention, the primers for amplifying the promoter region sequence of the gene are:
P9: 5'CATCTGTTCT CGGTGCACGACAGTGATT TTATACTCAT G 3'(SEQ ID NO:13)
P10: 5'GACGTTTCCA GATGCTCATCACCGAACCC GCTGCACTGT 3'(SEQ ID NO:14)

In one embodiment of the present invention, the primers for amplifying the coding region of the NCgl1089 gene or the coding region of the NCgl1089^{G508A} gene are:
P11: 5'ACAGTGCAGC GGGTTCGGTGATGAGCATCT GGAAACGTC 3'(SEQ ID NO:15)
P12: 5'CTTGATTTAATTGCGCCATCTCACCTCTTC TGTGGGCACG 3'(SEQ ID NO:16)

In one embodiment of the present invention, the NCgl1089 or NCgl1089^{G508A} fragment with its own promoter is amplified using the aforementioned P9/P12 as primers and the NCgl1089 gene promoter fragment and NCgl1089 or NCgl1089^{G508A} obtained by amplification as templates.

In one embodiment of the present invention, amplification is carried out using the aforementioned P7/P14 as primers, and the three fragments of upstream homologous fragment, the downstream homologous fragment and the NCgl1089 or NCgl1089^{G508A} with a self-promoter obtained by amplification as templates to obtain integrated homology arm fragments.

In one embodiment of the present invention, the PCR system used is as follows: 10×Ex Taq Buffer 5 µL, dNTP Mixture (2.5 mM each) 4 µL, Mg ²⁺ (25 mM) 4 µL, primers (10 pM) 2 µL each, Ex Taq (5U/µL) 0.25µL, total volume 50 µL. PCR amplification is performed as follows: pre-denaturation at 94°C for 5 min, denaturation at 94°C for 30s, annealing at 52°C for 30s, extension at 72°C for 90s (30 cycles), and overextension at 72°C for 10 min.

In one embodiment of the present invention, the NEBuider recombination system is used to assemble the shuttle plasmid PK18mobsacB and the integrated homology arm fragment to obtain an integrated plasmid.

In one embodiment of the present invention, the integrated plasmid is transfected into a host strain, and the NCgl1089 or NCgl1089^{G508A} gene is introduced into the genome of the host strain by means of homologous recombination.

In one embodiment of the present invention, the host strain is YP97158.

In one embodiment of the present invention, the host strain is a strain carrying the polynucleotide sequence shown in SEQ ID NO:2.

The present invention further provides a construction method of a Corynebacterium recombinant strain.

According to the present invention, the construction method includes the following steps:
amplifying the sequences of the coding region and promoter region of the NCgl1089 or NCgl1089^{G508A} gene, construct an overexpression plasmid vector, and transferring the vector into a host strain to implement the strain overexpressing the NCgl1089 or NCgl1089^{G508A} gene.

In one embodiment of the present invention, the primers for amplifying the NCgl1089 promoter fragment are:

P19:5'GCTTGCATGCCTGCAGGTCGACTCTAGAGGATCCCCGACAGTGATTTTATACTCATG
3'(SEQ ID NO:23)

P20: 5'GACGTTTCCA GATGCTCATCACCGAACCC GCTGCACTGT 3'(SEQ ID NO:24).

In one embodiment of the present invention, the primers for amplifying the NCgll089 or NCgl1089^{G508A} gene fragment are:
P21: 5'ACAGTGCAGC GGGTTCGGTGATGAGCATCT GGAAACGTC 3'(SEQ ID NO:25)
P22: 5'ATCAGGCTGAAAATCTTCTCTCATCCGCCAAAACTCACCTCTTCTGTGGGCACG 3'(SEQ ID NO:26).

In one embodiment of the present invention, by using the aforementioned P19/P22 as primers, and the NCgl1089 gene promoter fragment and NCgl1089 or NCgl1089 ^{G508A} obtained by amplification as templates, the NCgl1089 or NCgl1089^{G508A} fragment with its own promoter is obtained by overlap PCR amplification.

In one embodiment of the present invention, the PCR system: 10×Ex Taq Buffer 5 µL, dNTP Mixture (2.5 mM each) 4 µL, Mg ²⁺ (25 mM) 4 µL, primers (10 pM) 2 µL each, Ex Taq (5U/µL) 0.25µL, total volume 50 µL; the PCR amplification is performed as follows: pre-denaturation at 94°C for 5 min, denaturation at 94°C for 30s, annealing at 52°C for 30s, extension at 72°C for 90s (30 cycles), and overextension at 72°C for 10 min.

In one embodiment of the present invention, the NEBuider recombination system is used to assemble the shuttle plasmid pXMJ19 and the NCgl1089 or NCgl1089^{G508A} fragment with its own promoter to obtain an overexpression plasmid.

In one embodiment of the present invention, the host strain is YP97158.

In one embodiment of the present invention, the host strain is a strain carrying the polynucleotide sequence shown in SEQ ID NO:2.

The recombinant strain obtained by the present invention can be used alone in fermentation to produce L-lysine, and can also be mixed with other L-lysine-producing bacteria to produce L-lysine by fermentation.

The present invention further provides a method of producing L-lysine, the method including culturing a microorganism; and obtaining L-lysine from the culture.

The second aspect of the present invention:
The present invention provides L-amino acid-generating bacteria in which the expression of a polynucleotide coding the amino acid sequence of SEQ ID NO:35 is improved. The present invention further provides a method for producing L-amino acids by using the microorganism.

The amino acid sequence of SEQ ID NO: 35 is a protein coded by the gene NCgl0761.

The polynucleotide may code an amino acid sequence that is about 90% or more, about 92% or more, about 95% or more, about 97% or more, about 98% or more, about 99% or more sequence homology to the amino acid sequence of SEQ ID NO:35.

The bacteria have enhanced L-amino acids production capacity compared to unmodified strains.

In one embodiment of the present invention, the polynucleotide may include the nucleotide sequence of SEQ ID NO:33.

In one embodiment of the present invention, the polynucleotide coding the amino acid sequence of SEQ ID NO:35 has a point mutation such that leucine at position 31 of the amino acid sequence of SEQ ID NO:35 is replaced by different amino acids.

According to the present invention, it is preferred that leucine at position 31 is replaced by arginine.

According to the present invention, the amino acid sequence shown in SEQ ID NO: 35, wherein the amino acid sequence after the 31^{st} leucine is replaced by arginine is shown in SEQ ID NO: 36.

In one embodiment of the present invention, the polynucleotide sequence with point mutation is formed by the mutation of the 92^{nd} base of the polynucleotide sequence shown in SEQ ID NO: 33.

According to the present invention, the mutation includes the mutation of thymine (T) to guanine (G) at the 92^{nd} base of the polynucleotide sequence shown in SEQ ID NO: 33.

In one embodiment of the present invention, the polynucleotide sequence with point mutation includes the polynucleotide sequence shown in SEQ ID NO:34.

According to the present invention, the expression regulatory sequences of polynucleotides can be modified. Expression regulatory sequences control the expression of polynucleotides to which they are operably linked, and can include, for example, promoters, terminators, enhancers, silencers, and the like. Polynucleotides can have changes in the initiation codon.

In one embodiment of the present invention, the promoter is the promoter of the polynucleotide coding the amino acid sequence of SEQ ID NO:35 (NCgl0761 gene).

In one embodiment of the present invention, the microorganism belonging to the Corynebacterium is Corynebacterium glutamicum ATCC13869.

The present invention further provides a polynucleotide sequence, an amino acid sequence coded by the polynucleotide sequence, a recombinant vector including the polynucleotide sequence, and a recombinant strain containing the polynucleotide sequence.

According to the present invention, the polynucleotide sequence includes a polynucleotide coding a polypeptide including the amino acid sequence shown in SEQ ID NO: 35, wherein leucine at position 31 is replaced by different amino acids.

According to the present invention, preferably the leucine at position 31 is replaced by arginine.

According to the present invention, the amino acid sequence shown in SEQ ID NO: 35, wherein the amino acid sequence after the 31^{st} leucine is replaced by arginine is shown in SEQ ID NO: 36.

According to the present invention, preferably the polynucleotide sequence coding the amino acid sequence shown in SEQ ID NO:35 contains the polynucleotide sequence shown in SEQ ID NO:33.

In one embodiment of the present invention, the polynucleotide sequence is formed by the mutation of the 92^{nd} base of the polynucleotide sequence shown in SEQ ID NO: 33.

According to the present invention, the mutation includes the mutation of thymine (T) to guanine (G) at the 92^{nd} base of the polynucleotide sequence shown in SEQ ID NO: 33.

In one embodiment of the present invention, the polynucleotide sequence includes the polynucleotide sequence shown in SEQ ID NO:34.

According to the present invention, the amino acid sequence includes the amino acid sequence shown in SEQ ID NO:36.

According to the present invention, the recombinant vector is constructed by introducing the polynucleotide sequence into a plasmid.

In one embodiment of the present invention, the plasmid is the pK18mobsacB plasmid.

In another embodiment of the present invention, the plasmid is the pXMJ19 plasmid.

Specifically, the polynucleotide sequence and the plasmid can be constructed into a recombinant vector through the NEBuider recombination system.

According to the present invention, the recombinant strain contains the polynucleotide sequence.

As an embodiment of the present invention, the starting bacterium of the recombinant strain is YP97158.

As an embodiment of the present invention, the starting bacterium of the recombinant strain is ATCC 13869.

The present invention further provides a construction method of a recombinant strain generating L-amino acid.

According to the present invention, the construction method includes the following steps:
The polynucleotide sequence of the wild-type NCgl0761 shown in SEQ ID NO: 33 in the host strain is transformed, and the 92nd base is mutated to obtain a recombinant strain including the gene coding the mutant NCgl0761.

According to the construction method of the present invention, the transformation includes at least one of mutagenesis, PCR site-directed mutagenesis, and/or homologous recombination.

According to the construction method of the present invention, the mutation refers to the mutation of thymine (T) to guanine (G) at the 92nd base in SEQ ID NO: 33; specifically, the polynucleotide sequence including the gene coding the mutant NCgl0761 is shown in SEQ ID NO:34.

Further, the construction method includes the following steps:
(1) transforming the nucleotide sequence of the wild-type NCgl0761 gene as shown in SEQ ID NO: 33, so that the 92nd base is mutated to obtain a mutated NCgl0761 gene polynucleotide sequence;
(2) linking the mutated polynucleotide sequence with a plasmid to construct a recombinant vector;
(3) introducing the recombinant vector into a host strain to obtain the recombinant strain including the gene coding the mutant NCgl0761.

According to the construction method of the present invention, the step (1) includes: constructing a point-mutated NCgl0761 gene: according to the genome sequence of unmodified strain, synthesizing two pairs of primers P1 and P2 and P3 and P4 for amplifying the NCgl10761 gene fragment, introducing a point mutation into the wild-type NCgl0761 gene SEQ ID NO: 33 by PCR site-directed mutagenesis to obtain the point-mutated NCgl0761 gene nucleotide sequence SEQ ID NO: 34, denoted as NCgl0761^{L31R}.

In one embodiment of the present invention, the genome of the unmodified strain can be derived from ATCC13032 strain or ATCC13869, and its genome sequence can be obtained from the NCBI website.

In one embodiment of the present invention, in the step (1), the primers are:
P1:5'CAGTGCCAAGCTTGCATGCCTGCAGGTCGACTCTAGCTTGCAGCTAACCTATACCCC3'(SEQ ID NO:37)
P2:5'GCTTTTCAATATAATCACGTCCATCTGAGCCATC3'(SEQ ID NO:38)
P3:5'GATGGCTCAGATGGACGTGATTATATTGAAAAGC3'(SEQ ID NO:39)
P4:5'CAGCTATGACCATGATTACGAATTCGAGCTCGGTACCCCTCCCAAATAATTGCCGC3'(SEQ ID NO:40)

In one embodiment of the present invention, the PCR amplification is performed as follows: pre-denaturation at 94°C for 5 min, denaturation at 94°C for 30s, annealing at 52°C for 30s, and extension at 72°C for 40s (30 cycles), overextension at 72°C for 10min.

In one embodiment of the present invention, the overlap PCR amplification is performed as follows: pre-denaturation at 94°C for 5 min, denaturation at 94°C for 30s, annealing at 52°C for 30s, and extension at 72°C for 60s (30 cycles), overextension at 72°C for 10min.

According to the construction method of the present invention, the step (2) includes the construction of the recombinant plasmid, including: assembling the isolated and purified NCgl0761^{L31R} and pK18mobsacB plasmids through the NEBuider recombination system to obtain the recombinant plasmid.

According to the construction method of the present invention, the step (3) includes the construction of a recombinant strain, and the recombinant plasmid is transformed into a host strain to obtain a recombinant strain.

In one embodiment of the present invention, the transformation of step (3) is an electrotransformation method.

In one embodiment of the present invention, the host strain is YP97158.

In one embodiment of the present invention, the host strain is ATCC 13869.

In one embodiment of the present invention, the recombination is implemented by homologous recombination.

The fourth aspect of the present invention further provides a construction method of a recombinant strain generating L-amino acid.

According to the present invention, the construction method includes the following steps:
amplifying the upstream and downstream homology arm fragments of the NCgl0761 gene, the coding region of the NCgl0761 gene and its promoter region sequence, or, the coding region of the NCgl0761^{L31R} gene and its promoter region sequence, and introducing it into the genome of the host strain by homologous recombination NCgl0761 and NCgl0761^{L31R} genes to implement the strain overexpressing the NCgl0761 and NCgl0761^{L31R} genes.

In one embodiment of the present invention, the primers for amplifying the upstream homology arm fragment are: 
P7:5' CAGTGCCAAGCTTGCATGCCTGCAGGTCGACTCTAGAATGCGTTCTGGACTGAGG
3'(SEQ ID NO:43)
 P8:5'CCATCCATACCCCACTACATGTGCACCGAGAACAGATG 3'(SEQ ID NO:44)

In one embodiment of the present invention, the primers for amplifying the downstream homology arm fragments are:
P11:5'CTGAGCCGGAAACCTCACCC AGAATCAGATGGCGCAATTAAATC 3'(SEQ ID NO:47)

P12:5' CAGCTATGACCATGATTACGAATTCGAGCTCGGTACCCGCTATGACACCTTCAACGGATC
3'(SEQ ID NO:48)

In one embodiment of the present invention, the primers for amplifying the sequence of the coding region of the gene and its promoter region are:
P9:5'CATCTGTTCTCGGTGCACATGTAGTGGGGTATGGATGG 3'(SEQ ID NO:45)
P10:5'GATTTAATTGCGCCATCTGATTCTGGGTGAGGTTTCCGGCTCAG3'(SEQ ID NO:46).

In one embodiment of the present invention, the PCR system used is as follows: 10×Ex Taq Buffer 5 µL, dNTP Mixture (2.5 mM each) 4 µL, Mg ²⁺ (25 mM) 4 µL, primers (10 pM) 2 µL each, Ex Taq (5U/µL) 0.25µL, total volume 50 µL. PCR amplification is performed as follows: pre-denaturation at 94°C for 5 min, denaturation at 94°C for 30s, annealing at 52°C for 30s, extension at 72°C for 60s (30 cycles), and overextension at 72°C for 10 min.

In one embodiment of the present invention, the NEBuider recombination system is used to assemble the shuttle plasmid PK18mobsacB, the upper and lower homology arm fragments, the coding region of the gene and the promoter region fragment to obtain an integrated plasmid.

In one embodiment of the present invention, the integrated plasmid is transfected into a host strain, and the NCgl0761 or NCgl0761^{L31R} gene is introduced into the genome of the host strain by means of homologous recombination.

In one embodiment of the present invention, the host strain is YP97158.

In one embodiment of the present invention, the host strain is ATCC 13869.

In one embodiment of the present invention, the host strain is a strain carrying the polynucleotide sequence shown in SEQ ID NO:2.

The present invention further provides a construction method of a recombinant strain for producing L-amino acids.

According to the present invention, the construction method includes the following steps:
amplifying the coding region and the promoter region sequence of the NCgl0761 gene, or the coding region and the promoter region sequence of the NCgl0761^{L31R} gene, construct an overexpression plasmid vector, and transferring the vector into a host strain to implement the strain overexpressing the NCgl0761 and NCgl0761^{L31R} genes.

In one embodiment of the present invention, the primers for amplifying the sequence of the coding region of the gene and its promoter region are: 
P17:5' GCTTGCATGCCTGCAGGTCGACTCTAGAGGATCCCCATGTAGTGGGGTATGGATGG
3'(SEQ ID NO:53)
 P18:5' ATCAGGCTGAAAATCTTCTCTCATCCGCCAAAAC GTGAGGTTTC CGGCTCAG 3'(SEQ ID NO:54)

In one embodiment of the present invention, the PCR system is as follows: 10×Ex Taq Buffer 5 µL, dNTP Mixture (2.5 mM each) 4 µL, Mg ²⁺ (25 mM) 4 µL, primers (10 pM) 2 µL each, Ex Taq (5U/µL) 0.25µL, total volume 50 µL. PCR amplification is performed as follows: pre-denaturation at 94°C for 5 min, denaturation at 94°C for 30s, annealing at 52°C for 30s, extension at 72°C for 60s (30 cycles), and overextension at 72°C for 10 min.

In one embodiment of the present invention, the NEBuider recombination system is used to assemble the shuttle plasmid pXMJ19 and the NCgl0761 or NCgl0761^{L31R} fragment with its own promoter to obtain an overexpression plasmid.

In one embodiment of the present invention, the host strain is YP97158.

In one embodiment of the present invention, the host strain is ATCC 13869.

In one embodiment of the present invention, the host strain is a strain carrying the polynucleotide sequence shown in SEQ ID NO: 34.

The recombinant strain obtained by the present invention can be used alone in fermentation to produce an L-amino acid, and can also be mixed with other L- amino acid -producing bacteria to produce L- amino acid by fermentation.

The present invention further provides a method of producing an L-amino acid, the method including culturing the bacterium; and obtaining the L-amino acid from the culture.

The third aspect of the present invention:
The present invention provides an L-amino acid-generating bacterium, which has an improved expression of the polynucleotide coding the amino acid sequence of SEQ ID NO:63. According to the present invention, the improved expression is enhanced expression of the polynucleotide, or the polynucleotide coding the amino acid sequence of SEQ ID NO:63 has a point mutation, or the polynucleotide coding the amino acid sequence of SEQ ID NO:63 has a point mutation and the expression is enhanced.

The amino acid sequence of SEQ ID NO: 63 is a protein coded by the gene ptsS.

The bacteria have enhanced L-amino acid production capacity.

The bacteria having L-amino acid production capacity may be bacteria capable of accumulating the target L-amino acid in a medium in an amount of preferably 0.5 g/L or more, more preferably 1.0 g/L or more.

The polynucleotide may code an amino acid sequence that is about 90% or more, about 92% or more, about 95% or more, about 97% or more, about 98% or more, about 99% or more sequence homology to the amino acid sequence of SEQ ID NO:3.

In some embodiments of the present invention, the vector used is the pK18mobsacB plasmid, the pXMJ19 plasmid.

In a specific embodiment of the present invention, the polynucleotide coding the amino acid sequence of SEQ ID NO:63 may include the nucleotide sequence of SEQ ID NO:61.

In one embodiment of the present invention, the expression improvement refers to that the polynucleotide coding the amino acid sequence of SEQ ID NO: 63 has a point mutation such that the methionine at position 162 of the amino acid sequence of SEQ ID NO: 63 is replaced by different amino acids.

According to the present invention, preferably the methionine at position 162 is replaced by threonine.

According to the present invention, the amino acid sequence shown in SEQ ID NO:63, wherein the amino acid sequence after the 162^{nd} methionine is replaced by threonine is shown in SEQ ID NO:64.

In one embodiment of the present invention, the polynucleotide sequence with point mutation is formed by the mutation of the 485^{th} base of the polynucleotide sequence shown in SEQ ID NO: 61.

According to the present invention, the mutation includes the mutation of thymine (T) to cytosine (C) at the 485^{th} base of the polynucleotide sequence shown in SEQ ID NO: 61.

In one embodiment of the present invention, the polynucleotide sequence with point mutation includes the polynucleotide sequence shown in SEQ ID NO:62.

According to the present invention, the polynucleotide sequence is operably linked to a regulatory sequence.

In one specific embodiment of the present invention, the promoter is the promoter of the polynucleotide coding the amino acid sequence of SEQ ID NO:63 (ptsS gene).

In one embodiment of the present invention, the microorganism belonging to the Corynebacterium is Corynebacterium glutamicum ATCC13869. According to the present invention, the bacteria may also have other improvements related to increased L-amino acids production.

The present invention further provides a polynucleotide sequence, an amino acid sequence coded by the polynucleotide sequence, a recombinant vector including the polynucleotide sequence, and a recombinant strain containing the polynucleotide sequence.

According to the present invention, the polynucleotide sequence has an improved expression, the improvement including point mutation of the polynucleotide coding the polypeptide including the amino acid sequence shown in SEQ ID NO: 63 such that the methionine at position 162 of the amino acid sequence is replaced by different amino acids.

According to the present invention, preferably the methionine at position 162 is replaced by threonine.

According to the present invention, the amino acid sequence shown in SEQ ID NO:63, wherein the amino acid sequence after the 162^{nd} methionine is replaced by threonine is shown in SEQ ID NO:64.

According to the present invention, the polynucleotide sequence coding the amino acid sequence shown in SEQ ID NO:63 contains the polynucleotide sequence shown in SEQ ID NO:61.

In one embodiment of the present invention, the mutated polynucleotide sequence provided by the present invention is formed by the mutation of the 485^{th} base of the polynucleotide sequence shown in SEQ ID NO: 61.

According to the present invention, the mutation includes the mutation of thymine (T) to cytosine (C) at the 485^{th} base of the polynucleotide sequence shown in SEQ ID NO: 61.

In one embodiment of the present invention, the mutated polynucleotide sequence includes the polynucleotide sequence shown in SEQ ID NO:62.

According to the present invention, the replaced amino acid sequence includes the amino acid sequence shown in SEQ ID NO:64.

According to the present invention, the recombinant vector is constructed by introducing the polynucleotide sequence into a plasmid.

In one embodiment of the present invention, the plasmid is the pK18mobsacB plasmid.

In another embodiment of the present invention, the plasmid is the pXMJ19 plasmid.

Specifically, the polynucleotide sequence and the plasmid can be constructed into a recombinant vector through the NEBuider recombination system.

According to the present invention, the recombinant strain contains the polynucleotide sequence.

As an embodiment of the present invention, the starting bacterium of the recombinant strain is YP97158.

As an embodiment of the present invention, the starting bacterium of the recombinant strain is ATCC 13869.

The present invention further provides a construction method of a recombinant strain generating L-amino acid.

According to the present invention, the construction method includes the following steps:
transforming the polynucleotide sequence of the wild-type ptsS shown in SEQ ID NO: 61 in the host strain, and mutating the 485^{th} base to obtain a recombinant strain including the gene coding the mutant ptsS.

According to the construction method of the present invention, the transformation includes at least one of mutagenesis, PCR site-directed mutagenesis, and/or homologous recombination.

According to the construction method of the present invention, the mutation refers to the mutation of thymine (T) to cytosine (C) at the 485th base in SEQ ID NO: 61; specifically, the polynucleotide sequence including the gene coding the mutant ptsS is shown in SEQ ID NO:62.

Further, the construction method includes the following steps:
(1) transforming the nucleotide sequence of the wild-type ptsS gene as shown in SEQ ID NO: 61, so that the 485th base is mutated to obtain a mutated ptsS gene polynucleotide sequence;
(2) linking the mutated polynucleotide sequence with a plasmid to construct a recombinant vector;
(3) introducing the recombinant vector into a host strain to obtain the recombinant strain including the gene coding the mutant ptsS.

According to the construction method of the present invention, the step (1) includes: constructing a point-mutated ptsS gene: according to the genome sequence of unmodified strain, synthesizing two pairs of primers P1 and P2 and P3 and P4 for amplifying the ptsS gene fragment, a point mutation is introduced into the wild-type ptsS gene SEQ ID NO: 61 by PCR site-directed mutagenesis to obtain the point-mutated ptsS gene nucleotide sequence SEQ ID NO: 62, denoted as ptsS ^{T485C}.

In one embodiment of the present invention, the genome of the unmodified strain can be derived from ATCC13032 strain, and its genome sequence can be obtained from the NCBI website.

In one embodiment of the present invention, in the step (1), the primers are:
P1:5' CAGTGCCAAGCTTGCATGCCTGCAGGTCGACTCTAGGACACCTGAAG CACCTGC 3'(SEQ ID NO:65)
P2:5'GAGATGATCAACCTCACGGCATCTGCGC 3'(SEQ ID NO:66)
P3:5'GCGCAGATGCCGTGAGGTTGATCATCTC 3'(SEQ ID NO:67)
P4:5' CAGCTATGACCATGATTACCGAATTCGAGCTCGGTACCCGATGGACAGGTTTCATTCGC3'(SEQ ID NO:68)

In one embodiment of the present invention, the PCR amplification is performed as follows: pre-denaturation at 94°C for 5 min, denaturation at 94°C for 30s, annealing at 52°C for 30s, extension at 72°C for 40s (30 cycles), and overextension at 72°C for 10min.

In one embodiment of the present invention, the overlap PCR amplification is performed as follows: pre-denaturation at 94°C for 5 min, denaturation at 94°C for 30s, annealing at 52°C for 30s, and extension at 72°C for 90s (30 cycles), overextension at 72°C for 10min.

According to the construction method of the present invention, the step (2) includes the construction of the recombinant plasmid, including: assembling the isolated and purified ptsS^{M162T} and pK18mobsacB plasmids through the NEBuider recombination system to obtain the recombinant plasmid.

According to the construction method of the present invention, the step (3) includes the construction of a recombinant strain, and the recombinant plasmid is transformed into a host strain to obtain a recombinant strain.

vIn one embodiment of the present invention, the transformation of step (3) is an electrotransformation method.

In one embodiment of the present invention, the host strain is YP97158.

In one embodiment of the present invention, the recombination is implemented by homologous recombination.

The present invention further provides a construction method of a recombinant strain generating L-amino acid.

According to the present invention, the construction method includes the following steps:
Amplify the upstream and downstream homology arm fragments of the ptsS gene, the coding region and its promoter region sequence of the ptsS gene, or, amplify the coding region and its promoter region sequence of the PtsS or PtsS ^{M162T} gene, and the PtsS or PtsS ^{M162T} gene is introduced into the genome of the host strain by homologous recombination to obtain the strain overexpressing the PtsS or PtsS ^{M162T} gene.

In one embodiment of the present invention, the primers for amplifying the upstream homology arm fragment are: 
P7:5' CAGTGCCAAGCTTGCATGCCTGCAGGTCGACTCTAGAATGCGTTCTGGACTGAGG
3'(SEQ ID NO:71)
 P8:S'GTGACTCTACGCATCTTTGACAGTGCACCG AGAACAGATG 3'(SEQ ID NO:72)

In one embodiment of the present invention, the primers for amplifying the downstream homology arm fragments are:
P11:5'CACCACCACGATCCACAGACCCAGAATCAGATGGCGCAATTAAAT CAAG 3'(SEQ ID NO:75)

P12:5' CAGCTATGACCATGATTACGAATTCGAGCTCGGTACCCGCTATGACACCTTCAACGGATC
3'(SEQ ID NO:76)

In one embodiment of the present invention, the primers for amplifying the sequence of the coding region of the gene and its promoter region are:
P9:5'CATCTGTTCTCGGTGCACTGTCAAAGATGCGTA GAGTCAC 3'(SEQ ID NO:73)
P10:5'CTTGATTTAATTGCGCCATCTGATTCTGGGTCTGTGGATCGTGG TGGTG 3'(SEQ ID NO:74).

In one embodiment of the present invention, using the aforementioned P7-P12 as primers, and the three fragments of upstream homologous fragment, the downstream homologous fragment and the PtsS or PtsS^{M162T} with a self-promoter obtained by amplification as templates for amplification to obtain integrated homology arm fragments.

In one embodiment of the present invention, the PCR system used: lOxEx Taq Buffer 5 µL, dNTP Mixture (2.5 mM each) 4 µL, Mg ²⁺ (25 mM) 4 µL, primers (10 pM) 2 µL each, Ex Taq (5U/µL) 0.25µL, total volume 50 µL. PCR amplification is performed as follows: pre-denaturation at 94°C for 5 min, denaturation at 94°C for 30s, annealing at 52°C for 30s, extension at 72°C for 60s (30 cycles), and overextension at 72°C for 10 min.

In one embodiment of the present invention, the NEBuider recombination system is used to assemble the shuttle plasmid PK18mobsacB, the upper and lower homology arm fragments, the coding region of the gene and the promoter region fragment to obtain an integrated plasmid.

In one embodiment of the present invention, the integrated plasmid is transfected into a host strain, and the PtsS or PtsS^{M162T} gene is introduced into the genome of the host strain by means of homologous recombination.

In one embodiment of the present invention, the host strain is YP97158. In one embodiment of the present invention, the host strain is a strain carrying the polynucleotide sequence shown in SEQ ID NO:62.

The present invention further provides a construction method of a recombinant strain for producing L-amino acids.

According to the present invention, the construction method includes the following steps:

Amplify the coding region and the promoter region sequence of the PtsS gene, or the coding region and the promoter region sequence of the PtsS^{M162T} gene, construct an overexpression plasmid vector, and transfer the vector into a host strain to implement the strain overexpressing the PtsS and PtsS^{M162T} genes.

In one embodiment of the present invention, the primers for amplifying the sequence of the coding region of the gene and its promoter region are: 
P17:5'GCTTGCATGCCTGCAGGTCGACTCTAGAGGATCCCCTGTCAAAGATGCGTAGAGTCAC
3'(SEQ ID NO:81)
 P18:5'ATCAGGCTGAAAATCTTCTCTCATCCGCCAAAACGTCTGTGGATCGTGGTGGTG3'(SEQ ID NO:82)

In one embodiment of the present invention, the PCR system used: 10×Ex Taq Buffer 5 µL, dNTP Mixture (2.5 mM each) 4 µL, Mg ²⁺ (25 mM) 4 µL, primers (10 pM) 2 µL each, Ex Taq (5U/µL) 0.25µL, total volume 50 µL. PCR amplification is performed as follows: pre-denaturation at 94°C for 5 min, denaturation at 94°C for 30s, annealing at 52°C for 30s, extension at 72°C for 60s (30 cycles), and overextension at 72°C for 10 min.

In one embodiment of the present invention, the NEBuider recombination system is used to assemble the shuttle plasmid pXMJ19 and the PtsS or PtsS^{M162T} fragment with its own promoter to obtain an overexpression plasmid.

In one embodiment of the present invention, the host strain is YP97158.

In one embodiment of the present invention, the host strain is ATCC 13869.

In one embodiment of the present invention, the host strain is a strain carrying the polynucleotide sequence shown in SEQ ID NO:62.

The recombinant strain obtained by the present invention can be used alone in fermentation to produce an L-amino acid, and can also be mixed with other L- amino acid -producing bacteria to produce L- amino acid by fermentation.

The present invention further provides a method of producing an L-amino acid, the method including culturing the bacterium; and obtaining the L-amino acid from the culture.

The present invention is described in detail below:

The term "bacteria having L-amino acid production capacity" refers to the capacity to produce and accumulate the target L-amino acid in the medium and/or cells of the bacteria to such an extent that L-amino acids can be collected when the bacteria are cultured in the medium. The bacteria having L-amino acid production capacity may be bacteria capable of accumulating the target L-amino acid in the medium and/or the cells of the bacteria in a larger amount than that obtainable by the unmodified strain.

The term "unmodified strain" refers to a control strain that has not been modified in such a way that it has specific characteristics. That is, examples of unmodified strains include wild-type strains and parental strains.

The bacteria having L-amino acid production capacity may be bacteria capable of accumulating the target L-amino acid in a medium in an amount of preferably 0.5 g/L or more, more preferably 1.0 g/L or more.

Examples of L-amino acids include basic amino acids such as L-lysine, L-ornithine, L-arginine, L-histidine and L-citrulline; aliphatic amino acids such as L-isoleucine, L-alanine, L-valine, L-leucine, and glycine; amino acids that are hydroxy-monoaminocarboxylic acids, such as L-threonine and L-serine; cyclic amino acids, such as L-proline; aromatic amino acids such as L-phenylalanine, L-tyrosine and L-tryptophan; sulfur-containing amino acids such as L-cysteine, L-cystine and L-methionine; acidic amino acids such as L-glutamic acid and L-aspartic acid; and amino acids with an amide group on the side chain, such as L-glutamine and L-asparagine.

Specific examples of L-amino acids include L-glutamic acid, L-lysine, L-threonine, L-arginine, L-histidine, L-isoleucine, L-valine, L-leucine, L-phenylalanine, L-tyrosine, L-tryptophan and L-cysteine.

More specific examples of L-amino acids include L-glutamic acid, L-lysine, L-threonine and L-tryptophan. More specific examples of L-amino acids include L-glutamic acid, L-lysine.

In the present invention, unless otherwise specified, the term "amino acid" refers to L-amino acids. In the present invention, unless otherwise specified, the term "L-amino acid" refers to an L-amino acid in free form, a salt thereof, or a mixture thereof.

The term "homology" refers to the percent identity between two polynucleotides or two polypeptide modules. Sequence homology between one module and another can be determined by using methods known in the art. Such sequence homology can be determined, for example, by the BLAST algorithm.

In the present invention, the expression enhancement of polynucleotides refers to: by replacement or mutation of expression regulatory sequences, by introduction of mutations to the polynucleotide sequence, by an increase in the copy number of the polynucleotide through chromosomal insertion or introduction of a vector, or a combination thereof, and the like.

According to the present invention, the mutation refers to a change in the base/nucleotide of the site, and the mutation method can be selected from at least one of mutagenesis, PCR site-directed mutagenesis, and/or homologous recombination. In the present invention, PCR site-directed mutagenesis and/or homologous recombination are preferably employed.

The present invention can modify expression regulatory sequences of polynucleotides. Expression regulatory sequences control the expression of polynucleotides to which they are operably linked, and can include, for example, promoters, terminators, enhancers, silencers, and the like. Polynucleotides can have changes in the initiation codon. Polynucleotides can be incorporated into a specific site on the chromosome, thereby increasing copy number. Herein, a specific site may include, for example, a transposon site or an intergenic site. Additionally, polynucleotides can be incorporated into an expression vector, and the expression vector is introduced into a host cell to increase copy number.

In one embodiment of the present invention, the copy number is increased by incorporating a polynucleotide or a polynucleotide with a point mutation into a specific site on the chromosome of a microorganism.

In one embodiment of the present invention, a polynucleotide with a promoter sequence or a polynucleotide with a promoter sequence with a point mutation is incorporated into a specific site on the chromosome of the microorganism to overexpress the nucleic acid sequence.

In one embodiment of the present invention, a polynucleotide or a polynucleotide having a point mutation is incorporated into an expression vector, and the expression vector is introduced into a host cell to increase the copy number.

In one embodiment of the present invention, a polynucleotide with a promoter sequence or a polynucleotide with a promoter sequence with a point mutation is incorporated into an expression vector, and the expression vector is introduced into a host cell to overexpress the nucleic acid sequence.

The term "operably linked" refers to a functional linkage between a regulatory sequence and a polynucleotide sequence, whereby the regulatory sequence controls transcription and/or translation of the polynucleotide sequence. The regulatory sequence can be a strong promoter capable of increasing the expression level of the polynucleotide. Regulatory sequences may be promoters derived from microorganisms belonging to the Corynebacterium or may be promoters derived from other microorganisms. For example, the promoter can be a trc promoter, a gap promoter, a tac promoter, a T7 promoter, a lac promoter, a trp promoter, an araBAD promoter, or a cj7 promoter.

The term "vector" refers to a polynucleotide construct that contains a gene's regulatory and gene sequences and is configured to express a target gene in a suitable host cell. Alternatively, a vector may in turn refer to a polynucleotide construct containing sequences useful for homologous recombination, thus, due to the vector introduced into the host cell, the regulatory sequences of the endogenous gene on the genome of the host cell can be altered, or the target gene that can be expressed can be inserted into a specific site on the genome of the host. In this regard, the vector used in the present invention may further include a selectable marker to determine the introduction of the vector into the host cell or the insertion of the vector into the chromosome of the host cell. Selectable markers can include markers that confer a selectable phenotype, such as drug resistance, auxotrophy, resistance to cytotoxic agents, or expression of surface proteins. In an environment treated with such selective agents, transformed cells can be selected because only cells expressing the selectable marker can survive or display different phenotypic traits.

In some embodiments of the present invention, the vector used is the pK18mobsacB plasmid, the pXMJ19 plasmid.

The term "transformation" refers to the introduction of a polynucleotide into a host cell such that the polynucleotide is replicable as an extragenomic element or inserted into the genome of the host cell. The method of transforming the vector used in the present invention may include a method of introducing nucleic acid into cells. In addition, as disclosed in the related art, the electrical pulse method can be implemented according to the host cell.

In the present invention, the microorganisms belonging to the Corynebacterium can be Corynebacterium glutamicum, Brevibacterium flavum, Brevibacterium lactofermentum, Corynebacterium ammoniagenes, and Corynebacterium pekinense.

In one embodiment of the present invention, the microorganism belonging to the Corynebacterium is Corynebacterium glutamicum YP97158, preservation number: CGMCC No. 12856, preservation date: August 16, 2016, depository unit: China General Microbiological Culture Collection Center. No. 3, Yard 1, West Beichen Road, Chaoyang District, Beijing, Tel: 010-64807355, the strain is recorded in Chinese patent application CN106367432A (application date September 1, 2016, publication date February 1,2017).

In the present invention, the bacteria may also have other improvements related to increasing L-amino acid production, for example, the activity of enzymes such as glutamate dehydrogenase, aconitate hydratase, citrate synthase, methyl citrate synthase, pyruvate carboxylase, pyruvate dehydrogenase, pyruvate kinase, phosphoenolpyruvate synthase, phosphoglycerate mutase, phosphoglycerate kinase, glyceraldehyde-3-phosphate dehydrogenase, triose phosphate isomerase, fructose bisphosphate aldolase, glucose phosphate isomerase, 6-phosphogluconate dehydratase, 2-keto-3-deoxy-6-phosphogluconate aldolase, glucose dehydrogenase, gluconate kinase, aspartate kinase III, aspartate semialdehyde dehydrogenase, homoserine kinase, threonine synthase, dihydrodipicolinate synthase, dihydrodipicolinate reductase, m-diaminopimelate dehydrogenase, diaminopimelate decarboxylaseor the enhanced or reduced expression of genes, or the genes can be replaced by foreign genes.

Further, the microorganism may also have other improvements related to increased L-lysine production, for example, increased or decreased expression of genes associated with NADPH generation (e.g., a gene coding glucose dehydrogenase, a gene coding gluconate kinase, a gene coding aldehyde-3-phosphate dehydrogenase, a gene coding glucose-6-phosphate dehydrogenase, or a gene coding 6-phosphogluconate dehydrogenase) and/or other genes related to L-Lysine biosynthesis or secretion (e.g., a gene coding aspartate aminotransferase, a gene coding aspartate kinase, a gene coding aspartate semialdehyde dehydrogenase, a gene coding dihydrodipicolinate synthase, a gene coding dihydrodipicolinate reductase, a gene coding m-diaminopimelate dehydrogenase, a gene coding diaminopimelate decarboxylase, lysE), or may have genes replaced by foreign genes.

In the present invention, the mutation refers to a change in the base/nucleotide of the site, and the mutation method can be selected from at least one of methods such as mutagenesis, PCR site-directed mutagenesis, and/or homologous recombination. In the present invention, PCR site-directed mutagenesis and/or homologous recombination are preferably used.

In the present invention, the culturing of bacteria can be carried out in a suitable medium under culturing conditions known in the art. The medium may include: carbon sources, nitrogen sources, trace elements, and combinations thereof. During cultivation, the pH of the culture can be adjusted. In addition, the culturing may include preventing the production of air bubbles, for example, by using an antifoaming agent. In addition, culturing can include injecting a gas into the culture. The gas can include any gas capable of maintaining aerobic conditions of the culture. In the culture, the temperature of the culture can be 20 to 45°C. The resulting L-amino acids can be recovered from the culture by treating the culture with sulfuric acid or hydrochloric acid, etc., followed by a combination of methods such as anion exchange chromatography, concentration, crystallization, and isoelectric precipitation.

### BENEFICIAL EFFECT

The present invention takes Corynebacterium glutamicum YP97158 as the starting bacterium, weakens or knocks out the NCgl1089 gene, NCgl0761 gene and ptsS gene in its background, and finds that the product coded by the gene produce an impact on the L-amino acid production capacity. A point mutation is introduced into the coding sequence, or the number of copies of the gene is increased or the recombinant strain is obtained by overexpression, and the obtained strain has a significant L-amino acid production capacity, which is conducive to the production of high concentration of L-amino acid.

### BEST WAY TO IMPLEMENT THE INVENTION

The above and other features and advantages of the present invention are explained and illustrated in more detail below through the description of the embodiments of the present invention. It should be understood that the following embodiments are intended to illustrate the technical solutions of the present invention, but are not intended to limit the protection scope of the present invention defined by the claims and their equivalents.

Unless otherwise specified, the raw materials and reagents used in the following examples are all commercially available products, or can be prepared by known methods; the operations performed are known in the art, or are carried out according to the user manuals of commercially available products.

The basal medium used for culturing the strains in the following examples has the same composition, and correspondingly required sucrose, kanamycin or chloramphenicol and the like are added to this basal medium composition, and the basal medium composition is as follows:

| Ingredient | Recipe |
|---|---|
| Sucrose | 10g/L |
| Polypeptone | 10g/L |
| Beef extract | 10g/L |
| Yeast powder | 5g/L |
| Urea | 2g/L |
| Sodium chloride | 2.5g/L |
| Agar powder | 20g/L |
| pH | 7.0 |
| Incubation temperature | 32°C |

The preparation and conditions of SSCP electrophoresis PAGE described in the following examples are as follows:

| Ingredient | Dosage (Configure acrylamide at a final concentration of 8%) |
|---|---|
| 40 % Acrylamide | 8m1 |
| ddH₂O | 26m1 |
| Glycerin | 4m1 |
| 10*TBE | 2m1 |
| TEMED | 40ul |
| 10%AP | 600ul |
| Electrophoresis conditions | Put the electrophoresis tank in ice, use 1× TBE buffer, voltage 120V, electrophoresis time 10h |

### Example 1

### (1) Construction of transformation vector pK18-NCgl1089^{G508A} containing the coding region of NCgl1089 gene with point mutation

According to the genome sequence of wild-type Corynebacterium glutamicum ATCC13032 published by NCBI, two pairs of primers for amplifying the coding region sequence of the NCgl1089 gene were designed and synthesized, a point mutation was introduced in the coding region (SEQ ID NO: 1) of the NCgl1089 gene in the background of strain YP97158(preservation number: CGMCC No. 12856, preservation date: August 16, 2016, depository unit: China General Microbiological Culture Collection Center. No. 3, Yard 1, West Beichen Road, Chaoyang District, Beijing, Tel: 010-64807355, the strain was recorded in Chinese patent application CN106367432A (application date September 1, 2016, publication date February 1,2017)) by means of allelic replacement, the amino acid sequence corresponding to the coded protein was SEQ ID NO: 3, the 508^{th} of the nucleotide sequence of the NCgl1089 gene was changed from G to A (SEQ ID NO: 2: NCgl1089^{G508A}), and the 170^{th} of the amino acid sequence corresponding to the coded protein was changed from glutamic acid to lysine (SEQ ID NO: 4: NCgl1089E170K).

The primers were designed as follows (synthesized by Shanghai Invitrogen Company):

P1:5'CAGTGCCAAGCTTGCATGCCTGCAGGTCGACTCTAGCGCGTGGATCCACGCCAG
3'(SEQ ID NO:5)

P2:5'CAATGAGGGCTTTCGCCACCTCGCGGGC 3'(SEQ ID NO:6)
P3:5'GCCCGCGAGGTGGCGAAAGCCCTCATTG 3'(SEQ ID NO:7)

P4:5'CAGCTATGACCATGATTACGAATTCGAGCTCGGTACCCCATGCGTTGGCGATCTTC
3'(SEQ ID NO:8)

Construction method: Corynebacterium glutamicum ATCC13032 was used as a template to carry out PCR amplification with primers P1 and P2, P3 and P4, respectively.

PCR system: 10×Ex Taq Buffer 5µL, dNTP Mixture (2.5mM each) 4µL, Mg²⁺ (25mM) 4µL, primers (10µM) 2µL each, Ex Taq (5U/µL) 0.25µL, total volume 50µL.

The PCR amplification was performed as follows: pre-denaturation at 94°C for 5 min, denaturation at 94°C for 30s, annealing at 52°C for 30s, extension at 72°C for 40s (30 cycles), and overextension at 72°C for 10min to obtain two DNA fragments (NCgl1089Up and NCgl1089Down) with sizes of 724bp and 839bp and containing the coding region of the NCgl1089 gene, respectively.

After the above two DNA fragments were separated and purified by agarose gel electrophoresis, a fragment of about 1535 bp in length was amplified by overlap PCR using the above two DNA fragments as templates and P1 and P4 as primers.

PCR system: 10×Ex Taq Buffer 5µL, dNTP Mixture (2.5mM each) 4µL, Mg ²⁺ (25mM) 4µL, primers (10µM) 2µL each, Ex Taq (5U/µL) 0.25µL, total volume 50µL.

The PCR amplification was performed as follows: pre-denaturation at 94°C for 5 min, denaturation at 94°C for 30s, annealing at 52°C for 30s, extension at 72°C for 90s (30 cycles), and overextension at 72°C for 10 min.

This DNA fragment (NCgl1089^{G508A}) causes the guanine (G) at position 508 of the coding region of the YP97158 NCgl1089 gene to be changed to adenine (A), and finally the amino acid at position 170 of the coded protein was changed from glutamic acid (E) to lysine amino acid (K).

After the pK18mobsacB plasmid (purchased from Addgene) was digested with Xba I, the linearized pK18mobsacB plasmid and NCgl1089^{G508A} were separated and purified by agarose gel electrophoresis, and then assembled by the NEBuider recombination system to obtain the vector pK18-NCgl1089^{G508A}, which contains Kanamycin resistance marker. The vector pK18-NCgl1089^{G508A} was sent to a sequencing company for sequencing identification, and the vector pK18-NCgl1089^{G508A} containing the correct point mutation (G-A) was saved for future use.

### (2) Construction of engineering strain NCgl1089^{G508A} containing point mutation

Construction method: The allelic replacement plasmid pK18-NCgl1089^{G508A} was transformed into the patented strain YP97158 of L-lysine-producing bacteria by electric shock (for its construction method, please refer to WO2014121669A1; it was confirmed by sequencing that the coding region of the wild-type NCgl1089 gene was retained on the chromosome of this strain), the single colony produced by culture was identified by primer P1 and universal primer M13R respectively, and the strainthat could amplify a 1542bp size band was positive strain. The positive strains were cultured on the medium containing 15% sucrose, and the single colony produced by the culture was cultured on the medium containing kanamycin and without kanamycin, respectively, The strains that grew on the medium without kanamycin but did not grow on the medium containing kanamycin were further identified by PCR using the following primers (synthesized by Shanghai Invitrogen Company):
P5:5'GGTGATTGATGCATTATGCGC 3'(SEQ ID NO:9)
P6:5'CCTAGCCTTTCACCTCTTCTGT 3'(SEQ ID NO:10)

The above PCR amplification products were subjected to SSCP electrophoresis (the amplified fragment of plasmid pK18-NCgl1089^{G508A} was used as a positive control, the amplified fragment of YP97158 was used as a negative control, and water was used as a blank control) after high temperature denaturation and ice bath. Due to the different fragment structures and electrophoresis positions, the strains whose electrophoresis positions were inconsistent with those of the negative control fragment and were consistent with the positive control fragment were the strains with successful allelic replacement. The target fragment of the strain with successful allelic substitution was amplified again by PCR with primers P5 and P6, and was linked to the PMD19-T vector for sequencing. The successful allelic substitution of the strain was verified by sequence alignment of the mutated base sequence, and was named YPL-4-017.

### (3) Construction of engineering strains overexpressing NCgl1089 or NCgl1089^{G508A} gene on the genome

According to the genome sequence of wild-type Corynebacterium glutamicum ATCC13032 published by NCBI, four pairs of primers for amplifying the sequences of the upstream and downstream homology arm fragments, the coding region of the NCgl1089 gene, the coding region of the NCgl1089^{G508A} gene, and the promoter region of the gene were designed and synthesized, and the NCgll089 or NCgl1089^{G508A} gene was introduced into strain YP97158 by homologous recombination.

The primers were designed as follows (synthesized by Shanghai Invitrogen Company):
P7:5'CAGTGCCAAGCTTGCATGCCTGCAGGTCGACTCTAGAATGCGTTCTGGACTGAGG 3'(SEQ ID NO:11)
P8: 5'CATGAGTATA AAATCACTGT CGTGCACCGAG AACAGATG 3'(SEQ ID NO:12)
P9: S'CATCTGTTCT CGGTGCACGACAGTGATT TTATACTCAT G 3'(SEQ ID NO:13)
P10: 5'GACGTTTCCA GATGCTCATCACCGAACCC GCTGCACTGT 3'(SEQ ID NO:14)
P11: 5'ACAGTGCAGC GGGTTCGGTGATGAGCATCT GGAAACGTC 3'(SEQ ID NO:15)
P12: 5'CTTGATTTAATTGCGCCATCTCACCTCTTC TGTGGGCACG 3'(SEQ ID NO:16)
P13: S'CGTGCCCACAGAAGAGGTGAGAT GGCGCAATTA AATCAAG 3'(SEQ ID NO:17)

P14:5'CAGCTATGACCATGATTACGAATTCGAGCTCGGTACCCGCTATGACACCTTCAACGGA
TC 3'(SEQ ID NO:18)

Construction method: Corynebacterium glutamicum ATCC13032 was used as a template to carry out PCR amplification with primers P7/P8, P9/P10, P13/P14, respectively, to obtain an upstream homology arm fragment of 805bp, NCgl1089 gene promoter fragment of 318bp, and downstream homology arm fragment of 628bp; Corynebacterium glutamicum ATCC13032 and YPL-4-017 were then used as templates respectively, and P11/P12 were used as primers, and the NCgl1089 or NCgl1089^{G508A} gene fragment of 694bp was obtained by PCR amplification; P9/P12 was then used as primers, and NCgl1089 gene promoter fragment and NCgl1089 or NCgl1089^{G508A} were used as templates to obtain a 972bp fragment of NCgl1089 or NCgl1089^{G508A} with its own promoter; P7/P14 were then used as primers, and the above homology fragment, the lower homology fragment and the NCgl1089 or NCgl1089^{G508A} fragments with their own promoters were mixed as templates for amplification, and the integrated homology arm fragment was obtained.

After the PCR reaction, the amplified product was recovered by electrophoresis, and the required 2365bp DNA fragment was recovered by using a column DNA gel recovery kit (TIANGEN), and was assembled with the shuttle plasmid PK18mobsacB recovered by Xba I digestion through the NEBuider recombination system to obtain the integrated plasmid PK18mobsacB-NCgl1089 or PK18mobsacB-NCgl1089^{G508A}. The plasmid contained a kanamycin resistance marker, and the recombinant plasmid integrated into the genome can be obtained by kanamycin selection.

PCR system: 10×Ex Taq Buffer 5µL, dNTP Mixture (2.5mM each) 4µL, Mg²⁺ (25mM) 4µL, primers (10µM) 2µL each, Ex Taq (5U/µL) 0.25µL, total volume 50µL.

The PCR amplification was performed as follows: pre-denaturation at 94°C for 5 min, denaturation at 94°C for 30s, annealing at 52°C for 30s, extension at 72°C for 90s (30 cycles), and overextension at 72°C for 10 min.

The two integrated plasmids were electro-transformed into the patented strain YP97158 of L-lysine-producing bacteria respectively, and the single colony produced by the culture was identified by PCR through the P15/P16 primers, and the strain that could amplify a 1332bp size band was a positive strain, and those that fail to amplify the fragments were original strains. The positive strains were screened on the 15% sucrose medium and cultured on the medium containing kanamycin and without kanamycin, respectively. The strains that grew on the medium without kanamycin but did not grow on the medium containing kanamycin were further identified by PCR using P17/P18 primers, and the amplified strains with a size of 1187 bp were the strains whose genes were integrated into the YP97158 genome, which were named as YPL-4-018 (without point mutation) and YPL-4-019 (with point mutation) respectively.
P15:5'TCCAAGGAAGATACACGCC 3'(SEQ ID NO:19)
P16:5'CTGCGATTCC CAACGCATCT3'(SEQ ID NO:20)
P17:5'GAGCAGCCAA AACATGCAGC3'(SEQ ID NO:21)
P18:5'CGTTGGAATC TTGCGTTG 3'(SEQ ID NO:22)

### (4) Construction of engineering strains overexpressing NCgl1089 or NCgl1089^{G508A} gene on a plasmid

According to the genome sequence of wild-type Corynebacterium glutamicum ATCC13032 published by NCBI, two pairs of primers for amplifying the sequence of the coding region and the promoter region of NCgl1089 or NCgl1089^{G508A} gene were designed and synthesized. The primers were designed as follows (synthesized by Shanghai Invitrogen Company):

P19:5'GCTTGCATGCCTGCAGGTCGACTCTAGAGGATCCCCGACAGTGATTTTATACTCATG
3'(SEQ ID NO:23)

P20: 5'GACGTTTCCA GATGCTCATCACCGAACCC GCTGCACTGT 3'(SEQ ID NO:24)
P21: 5'ACAGTGCAGC GGGTTCGGTGATGAGCATCT GGAAACGTC 3'(SEQ ID NO:25)
P22 : 5'ATCAGGCTGAAAATCTTCTCTCATCCGCCAAAACTCACCTCTTCTGTGGGCACG 3'(SEQ ID NO:26)

Construction method: YPL-4-018 was used as a template, and the NCgl1089 promoter fragment of 378 bp was obtained by PCR with primers P19/P20; Wild-type Corynebacterium glutamicum ATCC13032 and YPL-4-017 were then used as templates respectively, and the NCgl1089 or NCgl1089^{G508A} gene fragment of 708 bp was obtained by PCR with primers P21/P22; the above promoter and gene fragment were used as templates, and the NCgl1089 or NCgl1089^{G508A} gene fragment of 1066bp with its own promoter was obtained by overlap PCR with primers P19/P22. The amplified product was recovered by electrophoresis, and the required 1066bp DNA fragment was recovered using a column DNA gel recovery kit, and was assembled with the shuttle plasmid pXMJ19 recovered by EcoR I digestion through the NEBuider recombination system to obtain the overexpression plasmid pXMJ19-NCgl1089 or pXMJ19-NCgl1089^{G508A}. The plasmid contained a chloramphenicol resistance marker, and the plasmid could be transformed into the strain by chloramphenicol screening.

PCR system: 10×Ex Taq Buffer 5µL, dNTP Mixture (2.5mM each) 4µL, Mg²⁺ (25mM) 4µL, primers (10pM) 2µL each, Ex Taq (5U/µL) 0.25µL, total volume 50µL.

The PCR amplification was performed as follows: pre-denaturation at 94°C for 5 min, denaturation at 94°C for 30s, annealing at 52°C for 30s, extension at 72°C for 90s (30 cycles), and overextension at 72°C for 10 min.

The pXMJ19-NCgl1089 or pXMJ19-NCgl1089^{G508A} were electro-transformed into the patented strain YP97158 of L-lysine-producing bacteria respectively, and the single colony produced by the culture was identified by M13 (-48) and P22 primers. The 1104 bp fragment was the transformed strain, which was named YPL-4-020 (without point mutation) or YPL-4-021 (with point mutation), respectively. The PCR amplified fragment containing a fragment of size 1104 bp was the transformed strain, which was named YPL-4-020 (without point mutation) or YPL-4-021 (containing a point mutation) respectively.

### (5) Construction of engineering strains that lacks the NCgl1089 gene on its genome

According to the genome sequence of Corynebacterium glutamicum ATCC13032 published by NCBI, two pairs of primers for amplifying the fragments at both ends of the coding region of the NCgl1089 gene were synthesized as upstream and downstream homology arm fragments. The primers were designed as follows (synthesized by Shanghai Yingjun company):

P23:5'CAGTGCCAAGCTTGCATGCCTGCAGGTCGACTCTAGCACCGCATTCCCTTCATGAT
3'(SEQ ID NO:27)

P24:5'ACGAATCCGCGCCTAGCCTTTTATCTACTTCCAAAAAACTGC 3'(SEQ ID NO:28)
P25:5'GCAGTTTTTTGGAAGTAGATAAAAGGCTAGGCGCGGATTCGT 3'(SEQ ID NO:29)

P26:5'CAGCTATGACCATGATTACGAATTCGAGCTCGGTACCCCGAGGGAAAGGATATCGA
3'(SEQ ID NO:30)

Corynebacterium glutamicum ATCC13032 was used as a template, PCR amplification was carried out with primers P23/P24 and P25/P26, respectively, to obtain an upstream homology arm fragment of 779bp and a downstream homology arm fragment of 800bp, and then the entire homology arm fragment of 1539bp was obtained by overlap PCR with primers P23/P26. After the PCR reaction, the amplified product was recovered by electrophoresis, the required 1539bp DNA fragment was recovered by a column DNA gel recovery kit, and was linked with the shuttle plasmid pk18mobsacB plasmid recovered by Xba I digestion through the NEBuider recombination system to obtain a knockout plasmid. This plasmid contained a kanamycin resistance marker.

The knockout plasmid was electro-transformed into the patented strain YP97158 of producing lysine, and the single colony produced by the culture was identified by PCR with the following primers (synthesized by Shanghai Yingjun Company) respectively:
P27:5'CACCGCATTCCCTTCATGAT 3'(SEQ ID NO:31)
P28:S'CGAGGGAAAGGATATCGA 3'(SEQ ID NO:32)

The strains with 1429bp and 2401bp bands amplified by the above PCR were positive strains, and the strain with 2401bp band amplified was the original strain. Positive strains were screened on 15% sucrose medium and cultured on kanamycin-containing and kanamycin-free medium, respectively. The strains that grew on the medium without kanamycin but did not grow on the medium containing kanamycin were further identified by PCR using P27/P28 primers, and the amplified strains with a size of 1429 bp were the genetic engineering strains in which the coding region of the NCgl1089 gene had been knocked out, which was named YPL-4-022.

### (6) L-Lysine Fermentation Experiment

The strains constructed in the examples and the original strain YP97158 were subjected to fermentation experiments in the BLBIO-5GC-4-H fermentor (purchased from Shanghai Bailun Biotechnology Co., Ltd.) with the medium shown in Table 1 and the control process shown in Table 2. Each strain was repeated three times, and the results were shown in Table 3.

**Table 1 Formula of fermentation medium**

| Ingredient | Recipe |
|---|---|
| Starch hydrolyzed sugar | 30g/L |
| Ammonium sulfate | 12g/L |
| Magnesium sulfate | 0.87g/L |
| Molasses | 20g/L |
| Acidified corn steep liquor | 3mL/L |
| Phosphoric acid | 0.4mL/L |
| Potassium chloride | 0.53g/L |
| Defoamer (2% GPE) | 4mL/L |
| Ferrous sulfate | 120mg/L |
| Manganese sulfate | 120mg/L |
| Niacinamide | 42mg/L |
| Calcium pantothenate | 6.3mg/L |
| Vitamin B l | 6.3mg/L |
| Copper and zinc salt solution | 0.6g/L |
| Biotin | 0.88mg/L |

**Table 2 Fermentation control process**

| Correction DO100% | Temperature 37°C, air volume 4L/min, speed 1000rpm, tank pressure Ompa, calibrate after 5min | | |
|---|---|---|---|
| Inoculation amount | 10% | Culture temperature°C | 37°C |
| pH | pH6.9±0.05 | Dissolved oxygen DO | 10-30% |
| Initial conditions | Temperature 37°C, pH6.9, Tank pressure OMpa, Air volume 3L/min, Speed 550rpm | | |
| Whole process control | Whole process control 1. When the dissolved oxygen was less than 30%, increase the speed by 750rpm→800fpm→air volume 4L/min→850rpm→950rpm; 2, Fermentation 6h lift tank pressure 0.01Mpa; 12h lift tank pressure 0.02Mpa→0.03Mpa→0.04Mpa→0.05Mpa | | |
| Residual sugar control | 0.1-0.2% before F12h; after F12h, combined with DO, it was required to control residual sugar 0.1-0.05% | | |
| Ammonia nitrogen control | 0.1-0.15 before F12h; F12-F32h 0.15-0.25; 0.1-0.15 after F32h | | |
| Feeding material | 25% ammonia water, 70% concentrated sugar, 50% ammonium sulfate,10% GPE | | |
| Fermentation cycle | Around 48h | | |

**Table 3 L-lysine fermentation experiment results**

| Strain | L-lysine production (g/l00ml) | OD(660nm) |
|---|---|---|
| YP97158 | 18.8 | 37.5 |
| YPL-4-017 | 19.1 | 37.3 |
| YPL-4-018 | 19.3 | 37.6 |
| YPL-4-019 | 19.5 | 37.8 |
| YPL-4-020 | 19.4 | 37.2 |
| YPL-4-021 | 19.8 | 36.5 |
| YPL-4-022 | 18.0 | 37.7 |

The results were shown in Table 3. Overexpression of the NCgl1089 gene, or point mutation NCgl1089^{G508A} and overexpression of the coding region of the NCgl 1089 gene in Corynebacterium glutamicum contributed to the improvement of L-lysine production, while weakening or knocking out the gene was not conducive to the accumulation of L-lysine.

### Example 2

### (1) Construction of transformation vector pK18-NCgl0761^{L31R} containing the coding region ofNCgl0761 gene with point mutation

According to the genome sequence of the Corynebacterium glutamicum ATCC13032 published by NCBI, two pairs of primers for amplifying the coding region sequence of the NCgl0761 gene were designed and synthesized, a point mutation was introduced in the coding region (SEQ ID NO: 33) of the NCgl0761 gene in the background of strain YP97158 (preservation number: CGMCC No. 12856, preservation date: August 16, 2016, depository unit: China General Microbiological Culture Collection Center. No. 3, Yard 1, West Beichen Road, Chaoyang District, Beijing, Tel: 010-64807355, the strain is recorded in Chinese patent application CN106367432A (application date September 1, 2016, publication date February 1,2017)) by means of allelic replacement, the amino acid sequence corresponding to the coded protein was SEQ ID NO: 35, the 92^{nd} of the nucleotide sequence of the NCgl0761 gene was changed from T to G (SEQ ID NO: 34), and the 31^{st} of the amino acid sequence corresponding to the coded protein was changed from leucine to arginine (SEQ ID NO: 36: NCgl0761^{L31R}). The primers were designed as follows (synthesized by Shanghai Invitrogen Company):
P1:5' CAGTGCCAAGCTTGCATGCCTGCAGGTCGACTCTAGCTTGCAGCTAACCTATACCCC3'(SEQ ID NO:37)
P2:5'GCTTTTCAATATAATCACGTCCATCTGAGCCATC3'(SEQ ID NO:38)
P3:5'GATGGCTCAGATGGACGTGATTATATTGAAAAGC3'(SEQ ID NO:39)
P4:5' CAGCTATGACCATGATTACGAATTCGAGCTCGGTACCCCTCCCAAATAATTGCCGC3'(SEQ ID NO:40)

Construction method: Corynebacterium glutamicum ATCC13032 was used as a template to carry out PCR amplification with primers P1 and P2, P3 and P4, respectively. PCR system: 10×Ex Taq Buffer 5µL, dNTP Mixture (2.5mM each) 4µL, Mg ²⁺ (25mM) 4µL, primers (10pM) 2µL each, Ex Taq (5U/µL) 0.25µL, total volume 50µL. The PCR amplification was performed as follows: pre-denaturation at 94°C for 5 min, denaturation at 94°C for 30s, annealing at 52°C for 30s, extension at 72°C for 40s, 30 cycles, and overextension at 72°C for 10min to obtain two DNA fragments (NCgl0761Up and NCgl0761Down) with sizes of 636bp and 681bp and containing the coding region of the NCgl0761 gene, respectively. After the above two DNA fragments were separated and purified by agarose gel electrophoresis, a fragment of about 1283 bp in length was amplified by overlap PCR using the above two DNA fragments as templates and P1 and P4 as primers.

PCR system: 10×Ex Taq Buffer 5µL, dNTP Mixture (2.5mM each) 4µL, Mg ²⁺ (25mM) 4µL, primers (10pM) 2µL each, Ex Taq (5U/µL) 0.25µL, total volume 50µL. The PCR amplification was performed as follows: pre-denaturation at 94°C for 5 min, denaturation at 94°C for 30s, annealing at 52°C for 30s, extension at 72°C for 60s, 30 cycles, and overextension at 72°C for 10 min.

This DNA fragment causes the thymine (T) at position 92 of the coding region of the YP97158 NCgl0761 gene to be changed to guanine (G), and finally the amino acid at position 31 of the coded protein was changed from leucine (L) to arginine (R). The DNA fragment was purified after agarose gel electrophoresis, and then ligated with the purified pK18mobsacB plasmid (purchased from Addgene Company, which was double digested with Xbal I/BamH I respectively) after double digestion with NEBuilder enzyme(purchased from NEB Company) at 50°C for 30 min. The single clone grown after the transformation of the ligation product was identified by PCR to obtain the positive vector pK18-NCgl0761^{L31R}, which contained the kanamycin resistance marker on this plasmid. The correctly digested vector pK18-NCgl0761^{L31R} was sent to a sequencing company for sequencing identification, and the vector pK18-NCgl0761^{L31R} containing the correct point mutation (T-G) was stored for future use.

### (2) Construction of engineering strain pK18-NCgl0761^{L31R} containing point mutation

Construction method: The allelic replacement plasmid pK18-NCgl0761^{L31R} was transformed into the patented strain YP97158 of L-lysine-producing bacteria by electric shock (for its construction method, please refer to WO2014121669A1; it was confirmed by sequencing that the coding region of the wild-type NCgl0761 gene was retained on the chromosome of this strain), the single colony produced by culture was identified by primer P1 and universal primer M13R respectively, and the strain that could amplify a 1369bp size band was positive strain. The positive strains were cultured on the medium containing 15% sucrose, and the single colony produced by the culture was cultured on the medium containing kanamycin and without kanamycin, respectively, The strains that grew on the medium without kanamycin but did not grow on the medium containing kanamycin were further identified by PCR using the following primers (synthesized by Shanghai Invitrogen Company):
P5:5'GAATGGAATAGGAGAATTGCG 3'(SEQ ID NO:41)
P6:5'CACCAGGCGTGGAAAGAG 3'(SEQ ID NO:42)

The above PCR amplification product 267bp was subjected to SSCP electrophoresis (the amplified fragment of plasmid pK18-NCgl0761^{L31R} was used as a positive control, the amplified fragment of YP97158 was used as a negative control, and water was used as a blank control) after high temperature denaturation at 95°C for 10min and ice bath for 5min. Due to the different fragment structures and electrophoresis positions, the strains whose electrophoresis positions were inconsistent with those of the negative control fragment and were consistent with the positive control fragment were the strains with successful allelic replacement. The fragment of the positive strain NCgl0761 was amplified by PCR with primers P5 and P6 again, and was linked to the PMD19-T vector for sequencing. Through sequence alignment, the strain with mutation (T-G) in the base sequence was a positive strain with successful allelic substitution, and was named YPL-4-029.

### (3) Construction of engineering strains overexpressing NCgl0761 or NCgl0761^{L31R} gene on the genome

According to the genome sequence of wild-type Corynebacterium glutamicum ATCC13032 published by NCBI, three pairs of primers for amplifying the sequences of the upstream and downstream homology arm fragments, the coding region of the NCgl0761 or NCgl0761^{L31R} gene, and the promoter region were designed and synthesized, and the NCgl0761 or NCgl0761^{L31R} gene was introduced into strain YP97158 by homologous recombination.

The primers were designed as follows (synthesized by Shanghai Invitrogen Company):

P7:5' CAGTGCCAAGCTTGCATGCCTGCAGGTCGACTCTAGAATGCGTTCTGGACTGAGG
3'(SEQ ID NO:43)

P8:5'CCATCCATACCCCACTACATGTGCACCGAGAACAGATG 3'(SEQ ID NO:44)
P9:5'CATCTGTTCTCGGTGCACATGTAGTGGGGTATGGATGG 3'(SEQ ID NO:45)
P10:5'GATTTAATTGCGCCATCTGATTCTGGGTGAGGTTTCCGGCTCAG3'(SEQ ID NO:46)
P11:5'CTGAGCCGGAAACCTCACCC AGAATCAGATGGCGCAATTAAATC 3'(SEQ ID NO:47)

P12:5' CAGCTATGACCATGATTACGAATTCGAGCTCGGTACCCGCTATGACACCTTCAACGGATC
3'(SEQ ID NO:48)

Construction method: Corynebacterium glutamicum ATCC13032 or YPL-4-029 was used as a template to carry out PCR amplification with primers P7/P8, P9/P10, P11/P12, respectively, to obtain upstream homology arm fragment of 802bp, NCgl0761 or NCgl0761^{L31R} gene and its promoter fragment of 737bp and the downstream homology arm fragment of 647bp. After the PCR reaction, the three amplified fragments were recovered by electrophoresis using a column DNA gel recovery kit (TIANGEN). The recovered three fragments were ligated with purified pK18mobsacB plasmid (purchased from Addgene Company, which was double digested with Xbal I/BamH I respectively) after double digestion with NEBuilder enzyme(purchased from NEB Company) at 50°C for 30 min, and the single clone grown after the transformation of the ligation product was identified by PCR with M13 primer to obtain the positive integrated plasmid and pK18mobsacB-NCgl0761 and pK18mobsacB-NCgl0761^{L31R}. The plasmid contained a kanamycin resistance marker, and the recombinant plasmid integrated into the genome can be obtained by kanamycin selection.

PCR system: 10×Ex Taq Buffer 5µL, dNTP Mixture (2.5mM each) 4µL, Mg ²⁺ (25mM) 4µL, primers (10pM) 2µL each, Ex Taq (5U/µL) 0.25µL, total volume 50µL. The PCR amplification was performed as follows: pre-denaturation at 94°C for 5 min, denaturation at 94°C for 30s, annealing at 52°C for 30s, extension at 72°C for 60s (30 cycles), and overextension at 72°C for 10 min.

The two correctly sequenced integrated plasmids were electro-transformed into the patented strain YP97158 of L-lysine-producing bacteria respectively, and the single colony produced by the culture was identified by PCR through the P13/P14 primers, and the strain that could amplify a 1325bp size band was positive strain, and those that fail to amplify the fragments were original strains. The positive strains were cultured on a medium containing 15% sucrose, and the single colony produced by the culture was further identified by PCR using P15/P16 primers. The amplified strains with a size of 1002 bp were positive strains with NCgl0761 or NCgl0761^{L31R} gene integrated into the genome of YP97158, which were named YPL-4-030 (without mutation point) and YPL-4-031 (with mutation point).
P13:5'TCCAAGGAAGATACACGCC 3'(SEQ ID NO:49)
P14:5'GCCTTGTTAATATCTTCCC 3'(SEQ ID NO:50)
P15:5'GGGAAGATATTAACAAGGC 3'(SEQ ID NO:51)
P16:5'CGTTGGAATCTTGCGTTG 3'(SEQ ID NO:52)

### (4) Construction of engineering strains overexpressing NCgl0761 or NCgl0761^{L31R} gene on a plasmid

According to the genome sequence of wild-type Corynebacterium glutamicum ATCC13032 published by NCBI, one pair of primers for amplifying the sequence of the coding region and the promoter region of NCgl0761 or NCgl0761^{L31R} gene were designed and synthesized. The primers were designed as follows (synthesized by Shanghai Invitrogen Company): 
P17:5' GCTTGCATGCCTGCAGGTCGACTCTAGAGGATCCCCATGTAGTGGGGTATGGATGG
3'(SEQ ID NO:53)
 P18:5' ATCAGGCTGAAAATCTTCTCTCATCCGCCAAAAC GTGAGGTTTCCGGCTCAG 3'(SEQ ID NO:54)

Construction method: Corynebacterium glutamicum ATCC13032 and YPL-4-029 were used as templates to carry out PCR amplification with primers P17/P18, respectively, to obtain NCgl0761 or NCgl0761^{L31R} gene and its promoter fragment of 737bp, and the amplified products were electrophoresed and purified using a column DNA gel recovery kit. The recovered DNA fragment and the shuttle plasmid pXMJ19 recovered by EcoR I digestion were ligated with NEBuilder enzyme (purchased from NEB company) at 50°C for 30 min, and the single clone grown after the transformation of the ligation product was identified by PCR with M13 primer to obtain the positive overexpression plasmid pXMJ19-NCgl0761 and pXMJ19-NCgl0761^{L31R}, and the plasmid was sent for sequencing. Because the plasmid contained the chloramphenicol resistance marker, it could be used to screen whether the plasmid was transformed into the strain by chloramphenicol.

PCR system: 10×Ex Taq Buffer 5µL, dNTP Mixture (2.5mM each) 4µL, Mg²⁺ (25mM) 4µL, primers (10pM) 2µL each, Ex Taq (5U/µL) 0.25µL, total volume 50µL.

The PCR amplification was performed as follows: pre-denaturation at 94°C for 5 min, denaturation at 94°C for 30s, annealing at 52°C for 30s, extension at 72°C for 60s (30 cycles), and overextension at 72°C for 10 min.

The correctly sequenced pXMJ19-NCgl0761 and pXMJ19-NCgl0761 ^{L31R} plasmids were electro-transformed into the patented strain YP97158 of L-lysine-producing bacteria, respectively, and the single colony produced by the culture were identified by PCR with primers M13/P18. The PCR amplified fragment containing a fragment of size 745 bp was the positive strain, which were named as YPL-4-032 (without mutation point) and YPL-4-033 (with mutation point).

### (5) Construction of engineering strains that lacks the NCgl0761 gene on its genome

According to the genome sequence of Corynebacterium glutamicum ATCC13032 published by NCBI, two pairs of primers for amplifying the fragments at both ends of the coding region of the NCgl0761 gene were synthesized as upstream and downstream homology arm fragments. The primers were designed as follows (synthesized by Shanghai Yingjun company): 
P19:5' CAGTGCCAAGCTTGCATGCCTGCAGGTCGACTCTAGGTATCTGGAGAGAAGAAGGAGC
3'(SEQ ID NO:55)
P20:5'GCCTTGTTAATATCTTCCCGAATACATGCCGCAATTCTCCTATTC3'(SEQ ID NO:56)
P21:5'GAGAATTGCGGCATGTATTCGGGAAGATATTAACAAGGC 3'(SEQ ID NO:57)

P22:5' CAGCTATGACCATGATTACGAATTCGAGCTCGGTACCCCCGCAGATTACTAAGGCTG
3'(SEQ ID NO:58)

Construction method: Corynebacterium glutamicum ATCC13032 was used as a template, PCR amplification was carried out with primers P19/P20 and P21/P22, respectively, to obtain an upstream homology arm fragment of 658bp and a downstream homology arm fragment of 716bp, and then the entire homology arm fragment of 1335bp was obtained by overlap PCR with primers P19/P22. The amplified product was electrophoresed and purified using a column DNA gel recovery kit, and the recovered DNA fragment was ligated with purified pK18mobsacB plasmid (purchased from Addgene Company, which was double digested with Xbal IIBamH I respectively) after double digestion with NEBuilder enzyme(purchased from NEB Company) at 50°C for 30 min, and the single clone grown after the transformation of the ligation product was identified by PCR with M13 primer to obtain the positive knockout vector pK18-ΔNCgl0761, which was sent for sequencing. This plasmid contained kanamycin resistance as a selectable marker.

PCR system: 10×Ex Taq Buffer 5µL, dNTP Mixture (2.5mM each) 4µL, Mg ²⁺ (25mM) 4µL, primers (10pM) 2µL each, Ex Taq (5U/µL) 0.25µL, total volume 50µL.

The PCR amplification was performed as follows: pre-denaturation at 94°C for 5 min, denaturation at 94°C for 30s, annealing at 52°C for 30s, extension at 72°C for 90s (30 cycles), and overextension at 72°C for 10 min.

The correctly sequenced knockout plasmid pK18-ΔNCgl0761 was electro-transformed into the patented strain YP97158 of producing lysine, and the single colony produced by the culture was identified by PCR with the following primers (synthesized by Shanghai Yingjun Company):
P23:5'GTATCTGGAGAGAAGAAGGAGC 3'(SEQ ID NO:59)
P24:5'CCGCAGATTACTAAGGCTG 3'(SEQ ID NO:60)

The strains with 1374bp and 1520bp bands simultaneously amplified by the above PCR were positive strains, and the strain with 1520bp band only amplified was the original strains. Positive strains were screened on 15% sucrose medium and cultured on kanamycin-containing and kanamycin-free medium, respectively. The strains that grew on the medium without kanamycin but did not grow on the medium containing kanamycin were further identified by PCR using P23/P24 primers, and the amplified strains with a size of 1374 bp were the positive strain in which the coding region of the NCgl0761 gene had been knocked out. The positive strain NCgl0761 fragment was amplified by PCR with P23/P24 primers again, and ligated to the PMD19-T vector for sequencing. The correctly sequenced strain was named YPL-4-034.

### (6) L-Lysine Fermentation Experiment

The strains constructed in the examples and the original strain YP97158 were subjected to fermentation experiments in the BLBIO-5GC-4-H fermentor (purchased from Shanghai Bailun Biotechnology Co., Ltd.) with the medium shown in Table 4 and the control process shown in Table 5. Each strain was repeated three times, and the results were shown in Table 6.

**Table 4 Formula of fermentation medium**

| Ingredient | Recipe |
|---|---|
| Starch hydrolyzed sugar | 30g/L |
| Ammonium sulfate | 12g/L |
| Magnesium sulfate | 0.87g/L |
| Molasses | 20g/L |
| Acidified corn steep liquor | 3mL/L |
| Phosphoric acid | 0.4mL/L |
| Potassium chloride | 0.53g/L |
| Defoamer (2% GPE) | 4mL/L |
| Ferrous sulfate | 120mg/L |
| Manganese sulfate | 120mg/L |
| Niacinamide | 42mg/L |
| Calcium pantothenate | 6.3mg/L |
| Vitamin B l | 6.3mg/L |
| Copper and zinc salt solution | 0.6g/L |
| Biotin | 0.88mg/L |

**Table 5 Fermentation control process**

| Correction DO100% | Temperature 37°C, air volume 4L/min, speed 1000rpm, tank pressure Ompa, calibrate after 5min | | |
|---|---|---|---|
| Inoculation amount | 10% | Culture temperature°C | 37°C |
| pH | pH6.9±0.05 | Dissolved oxygen DO | 10-30% |
| Initial conditions | Temperature 37°C, pH6.9, Tank pressure OMpa, Air volume 3L/min, Speed 550rpm | | |
| Whole process control | Whole process control 1. When the dissolved oxygen was less than 30%, increase the speed by 750rpm→800rpm→air volume | | |
| | 4L/min→850rpm→950rpm; 2, Fermentation 6h lift tank pressure 0.01Mpa; 12h lift tank pressure 0.02Mpa→0.03Mpa→0.04Mpa→0.05Mpa | | |
| Residual sugar control | 0.1-0.2% before F12h; after F12h, combined with DO, it was required to control residual sugar 0.1-0.05% | | |
| Ammonia nitrogen control | 0.1-0.15 before F12h; Fl2-F32h 0.15-0.25; 0.1-0.15 after F32h | | |
| Feeding material | 25% ammonia water, 70% concentrated sugar, 50% ammonium sulfate,10% GPE | | |
| Fermentation cycle | Around 48h | | |

**Table 6 L-lysine fermentation experiment results**

| Strain | L-lysine production (g/l00ml) | Transformation rate (%) |
|---|---|---|
| YP97158 | 18.9 | 65.2 |
| YPL-4-029 | 19.6 | 65.8 |
| YPL-4-030 | 19.4 | 65.6 |
| YPL-4-031 | 19.8 | 66.0 |
| YPL-4-032 | 19.3 | 65.7 |
| YPL-4-033 | 19.7 | 66.2 |
| YPL-4-034 | 18.0 | 63.8 |

The results were shown in Table 6. Overexpression of the NCgl0761 gene, or point mutation NCgl0761^{L31R} and overexpression of the coding region of the NCgl0761 gene in Corynebacterium glutamicum contributed to the improvement of L-lysine production and transformation rate, while weakening or knocking out the gene was not conducive to the accumulation of L-lysine, and at the same time reduced the transformation rate.

(7) The NCgl0761 gene was introduced into the glutamic acid-producing strain and overexpressed, or the coding region of the NCgl0761 gene was subjected to point mutation NCgl0761^{L31R} and overexpression, and fermentation experiments were carried out.

According to the method of Examples (1)-(5), the same primers and experimental conditions were used, and Corynebacterium ATCC13869 was used as the starting bacterium, and the bacterium of ATCC 13869 was used as the expression bacterium to obtain a glutamic acid-producing engineering strain of pK18-NCgl0761^{L31R} containing the point mutation(YPG-001), a glutamic acid-producing engineering strain overexpressing the NCgl0761(YPG-002) or NCgl0761^{L31R}(YPG-003) gene on the genome, and a glutamic acid-producing engineering strain overexpressing NCgl0761 (YPG-004) or NCgl0761^{L31R}(YPG-005) gene on plasmids, and a glutamic acid-producing engineering strain lacking the NCgl0761 gene on the genome(YPG-006).

The strains constructed in the examples and the original strain ATCC 13869 were subjected to fermentation experiments in the BLBIO-5GC-4-H fermentor (purchased from Shanghai Bailun Biotechnology Co., Ltd.) with the medium shown in Table 7 and the control process shown in Table 8. Each strain was repeated three times, and the results were shown in Table 9.

**Table 7 Formula of fermentation medium**

| Reagent name | Proportion |
|---|---|
| Glucose | 5.0g/L |
| Phosphoric acid | 0.38g/L |
| Magnesium sulfate | 1.85g/L |
| Potassium chloride | 1.6g/L |
| Biotin | 550ug/L |
| Vitamin B1 | 300µg/L |
| Ferrous sulfate | 10mg/L |
| Manganese sulfate | 10g/dl |
| KH₂PO₄ | 2.8g/L |
| Vitamin C | 0.75mg/L |
| Vitamin B12 | 2.5ug/L |
| Para aminobenzoic acid | 0.75mg/L |
| defoamer | 0.0015ml/dl |
| Betaine | 1.5g/L |
| Cane molasses | 7ml/L |
| Corn syrup | 77ml/L |
| Aspartic acid | 1.7g/L |
| Hair powder | 2g/L |

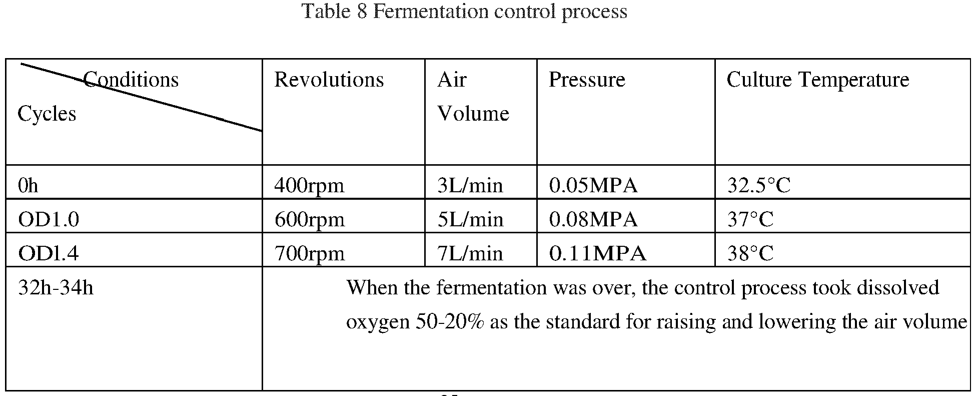

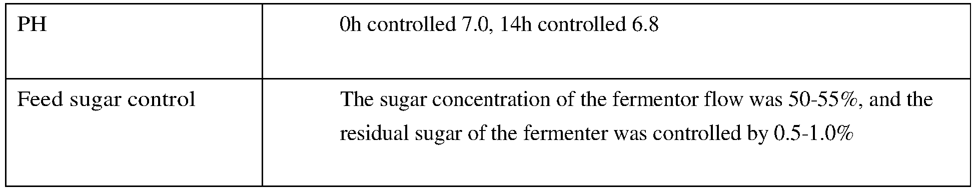

**Table 9 L-glutamic acid fermentation experiment results**

| Strain | L-glutamic acid production (g/l) | Transformation rate (%) |
|---|---|---|
| ATCC13869 | 101.0 | 45.8 |
| YPG-001 | 106.5 | 46.3 |
| YPG-002 | 106.9 | 46.6 |
| YPG-003 | 106.2 | 46.2 |
| YPG-004 | 106.6 | 46.5 |
| YPG-005 | 105.8 | 46.2 |
| YPG-006 | 97.5 | 45.3 |

The results were shown in Table 9. Overexpression of the NCgl0761 gene, or point mutation NCgl0761^{L31R} and overexpression of the coding region of the NCgl0761 gene in Corynebacterium glutamicum contributed to the improvement of L-lysine production and transformation rate, while weakening or knocking out the gene was not conducive to the accumulation of L-lysine, and at the same time reduced the transformation rate.

### Example 3

### (1)Construction of transformation vector pK18-ptsS^{M162T} containing the coding region of ptsS gene with point mutation

According to the genome sequence of wild-type Corynebacterium glutamicum ATCC13032 published by NCBI, two pairs of primers for amplifying the coding region sequence of the ptsS gene were designed and synthesized, a point mutation was introduced in the coding region (SEQ ID NO: 61) of the ptsS gene in the background of strain YP97158 (preservation number: CGMCC No. 12856, preservation date: August 16, 2016, depository unit: China General Microbiological Culture Collection Center. No. 3, Yard 1, West Beichen Road, Chaoyang District, Beijing, Tel: 010-64807355, the strain is recorded in Chinese patent application CN106367432A (application date September 1, 2016, publication date February 1,2017)) by means of allelic replacement, the amino acid sequence corresponding to the coded protein was SEQ ID NO: 63, the 485th of the nucleotide sequence of the ptsS gene was changed from thymine T to cytosine C (SEQ ID NO: 62), and the 162nd of the amino acid sequence corresponding to the coded protein was changed from methionine to threonine (SEQ ID NO: 64: ptsS^{M162T}). The primers were designed as follows (synthesized by Shanghai Invitrogen Company):
P1:5' CAGTGCCAAGCTTGCATGCCTGCAGGTCGACTCTAGGACACCTGAAGCACCTGC 3'(SEQ ID NO:65)
P2:5'GAGATGATCAACCTCACGGCATCTGCGC 3'(SEQ ID NO:66)
P3:5'GCGCAGATGCCGTGAGGTTGATCATCTC 3'(SEQ ID NO:67)
P4:5' CAGCTATGACCATGATTACGAATTCGAGCTCGGTACCCGATGGACAGGTTTCATTCGC3'(SEQ ID NO:68)

Construction method: Corynebacterium glutamicum ATCC13032 was used as a template, PCR amplification was carried out with primers P1 and P2, P3 and P4, respectively. PCR system: 10×Ex Taq Buffer 5µL, dNTP Mixture (2.5mM each) 4µL, Mg ²⁺ (25mM) 4µL, primers (10pM) 2µL each, Ex Taq (5U/µL) 0.25µL, total volume 50µL,The PCR amplification was performed as follows: pre-denaturation at 94°C for 5 min, (denaturation at 94°C for 30s, annealing at 52°C for 30s, extension at 72°C for 40s, 30 cycles), and overextension at 72°C for 10 min, two DNA fragments (ptsS Up and ptsS Down) containing the coding region of the ptsS gene were obtained with sizes of 666 bp and 703 bp, respectively. After the above two DNA fragments were separated and purified by agarose gel electrophoresis, the above two DNA fragments were used as templates, and P1 and P4 were used as primers, and a fragment with a length of 1341 bp was amplified by Overlap PCR.

PCR system: 10×Ex Taq Buffer 5µL, dNTP Mixture (2.5mM each) 4µL, Mg²⁺ (25mM) 4µL, primers (10pM) 2µL each, Ex Taq (5U/µL) 0.25µL, total volume 50µL. The PCR amplification was performed as follows: pre-denaturation at 94°C for 5 min (denaturation at 94°C for 30s, annealing at 52°C for 30s, extension at 72°C for 90s, 30 cycles), and overextension at 72°C for 10min.

This DNA fragment changed the thymine (T) at position 485 of the coding region of the YP97158ptsS gene into cytosine (C), and finally caused the 162nd amino acid of the coded protein to change from methionine (M) to threonine (T). This DNA fragment was purified by agarose gel electrophoresis, and was ligated with the pK18mobsacB plasmid purified after double digestion (purchased from Addgene, and double digestion with Xbal I/BamH I respectively) with NEBuilder enzyme at 50°C for 30 min. After the transformation of the ligation product, the monoclonal PCR was used to identify the vector pK18-ptsS^{M162T}, which contained a kanamycin resistance marker. The correctly digested vector pK18-ptsS^{M162T} was sent to a sequencing company for sequencing identification, and the vector pK18-ptsS^{M162T} containing the correct point mutation (T-C) was stored for future use.

### (2) Construction of engineering strain ptsS^{M162T} containing point mutation

Construction method: The allelic replacement plasmid pK18-ptsS^{M162T} was transformed into the patented strain YP97158 of L-lysine-producing bacteria by electric shock (for its construction method, please refer to WO2014121669A1; it was confirmed by sequencing that the coding region of the wild-type ptsS gene was retained on the chromosome of this strain, the single colony produced by culture was identified by primer P1 and universal primer M13R respectively, and the strain that could amplify a 1393bp size band was positive strain. The positive strains were cultured on the medium containing 15% sucrose, and the single colony produced by the culture was cultured on the medium containing kanamycin and without kanamycin, respectively, the strains that grew on the medium without kanamycin but did not grow on the medium containing kanamycin were further identified by PCR using the following primers (synthesized by Shanghai Invitrogen Company):
P5:5'CCACATTGGCATTTCGCC 3'(SEQ ID NO:69)
P6:5'CGCTGATTCCAATCTTGG 3'(SEQ ID NO:70)

The above PCR amplification product 311bp was subjected to sscp electrophoresis (the amplified fragment of plasmid pK18-ptsS^{M162T} was used as a positive control, the amplified fragment of YP97158 was used as a negative control, and water was used as a blank control) after high temperature denaturation at 95°C for 10min and ice bath for 5min. Due to the different fragment structures and electrophoresis positions, the strains whose electrophoresis positions were inconsistent with those of the negative control fragment and were consistent with the positive control fragment were the strains with successful allelic replacement. The fragment of the positive strain ptsS was amplified by PCR with primers P5 and P6 again, and was linked to the PMD19-T vector for sequencing. Through sequence alignment, the strain with mutation (A-G) in the base sequence was a positive strain with successful allelic substitution, and was named YPL-4-035.

### (3) Construction of engineering strains overexpressing PtsS or PtsS^{M162T} gene on the genome

According to the genome sequence of wild-type Corynebacterium glutamicum ATCC13032 published by NCBI, three pairs of primers for amplifying the sequences of the upstream and downstream homology arm fragments, the coding region of the PtsS or PtsS^{M162T} gene, and the promoter region were designed and synthesized, and the PtsS^{M162T} gene was introduced into strain YP97158 by homologous recombination.

The primers were designed as follows (synthesized by Shanghai Invitrogen Company):

P7:5' CAGTGCCAAGCTTGCATGCCTGCAGGTCGACTCTAGAATGCGTTCTGGACTGAGG
3'(SEQ ID NO:71)

P8:S'GTGACTCTACGCATCTTTGACAGTGCACCG AGAACAGATG 3'(SEQ ID NO:72)
P9:5'CATCTGTTCTCGGTGCACTGTCAAAGATGCGTA GAGTCAC 3'(SEQ ID NO:73)
P10:5'CTTGATTTAATTGCGCCATCTGATTCTGGGTCTGTGGATCGTGG TGGTG 3'(SEQ ID NO:74)
P11:5'CACCACCACGATCCACAGACCCAGAATCAGATGGCGCAATTAAAT CAAG 3'(SEQ ID NO:75)

P12:5' CAGCTATGACCATGATTACGAATTCGAGCTCGGTACCCGCTATGACACCTTCAACGGATC
3'(SEQ ID NO:76)

Construction method: Corynebacterium glutamicum ATCC13032 or YPL-4-035 was used as a template to carry out PCR amplification with primers P7/P8, P9/P10, P11/P12, respectively, to obtain upstream homology arm fragment of 802bp, PtsS or PtsS^{M162T} gene and its promoter fragment of 2354bp and the downstream homology arm fragment of 647bp. After the PCR reaction, the three amplified fragments were recovered by electrophoresis using a column DNA gel recovery kit (TIANGEN). The recovered three fragments were ligated with purified pK18mobsacB plasmid (purchased from Addgene Company, which was double digested with Xbal I/BamH I respectively) after double digestion with NEBuilder enzyme(purchased from NEB Company) at 50°C for 30 min. The single clone grown after the transformation of the ligation product was identified by PCR to obtain the positive integrated plasmid and pK18mobsacB-PtsS or PtsS^{M162T}and pK18mobsacB-PtsS^{M162T}. The plasmid contained a kanamycin resistance marker, and the recombinant plasmid integrated into the genome can be obtained by kanamycin selection.

PCR system: 10×Ex Taq Buffer 5µL, dNTP Mixture (2.5mM each) 4µL, Mg ²⁺ (25mM) 4µL, primers (10pM) 2µL each, Ex Taq (5U/µL) 0.25µL, total volume 50µL. The PCR amplification was performed as follows: pre-denaturation at 94°C for 5 min, denaturation at 94°C for 30s, annealing at 52°C for 30s, extension at 72°C for 60s (30 cycles), and overextension at 72°C for 10 min.

The two correctly sequenced integrated plasmids were electro-transformed into the patented strain YP97158 of L-lysine-producing bacteria respectively, and the single colony produced by the culture was identified by PCR through the P13/P14 primers, and the strain that could amplify a 1298bp size band was positive strain, and those that fail to amplify the fragments were original strains. The positive strains were cultured on a medium containing 15% sucrose, and the single colony produced by the culture was further identified by PCR using P15/P16 primers. The amplified strains with a size of 1133 bp were positive strains with PtsS or PtsS ^{M162T} gene integrated into the genome of YP97158, which were named YPL-4-036 (without mutation point) and YPL-4-037 (with mutation point).
P13:5'TCCAAGGAAGATACACGCC 3'(SEQ ID NO:77)
P14:5'GTGGAAAGATTGTGGTGGC 3'(SEQ ID NO:78)
P15:5'CATCCAGACTTTGGCGATC 3'(SEQ ID NO:79)
P16:5'CGTTGGAATCTTGCGTTG 3'(SEQ ID NO:80)

### (4) Construction of engineering strains overexpressing PtsS or PtsS^{M162T} gene on a plasmid

According to the genome sequence of wild-type Corynebacterium glutamicum ATCC13032 published by NCBI, one pair of primers for amplifying the sequence of the coding region and the promoter region of PtsS or PtsS^{M162T} gene were designed and synthesized. The primers were designed as follows (synthesized by Shanghai Invitrogen Company): 
P17:5' GCTTGCATGCCTGCAGGTCGACTCTAGAGGATCCCCTGTCA AAGATG
CGTAGAGTCAC 3'(SEQ ID NO:81)
 P18:5' ATCAGGCTGAAAATCTTCTCTCATCCGCCAAAACGTCTGTGGATCGTGGTGGTG3'(SEQ ID NO:82)

Construction method: ATCC13032 and YPL-4-035 were used as templates to carry out PCR amplification with primers P17/P18, to obtain PtsS or PtsS^{M162T} gene and its promoter fragment of 2354bp, and the amplified products were electrophoresed and purified using a column DNA gel recovery kit. The recovered DNA fragment and the shuttle plasmid pXMJ19 recovered by EcoR I digestion were ligated with NEBuilder enzyme (purchased from NEB company) at 50°C for 30 min, and the single clone grown after the transformation of the ligation product was identified by PCR with M13 primer to obtain the positive overexpression plasmid pXMJ19-PtsS and pXMJ19-PtsS^{M162T}, and the plasmid was sent for sequencing. Because the plasmid contained the chloramphenicol resistance marker, it could be used to screen whether the plasmid was transformed into the strain by chloramphenicol.

PCR system: 10×Ex Taq Buffer 5µL, dNTP Mixture (2.5mM each) 4µL, Mg²⁺ (25mM) 4µL, primers (10pM) 2µL each, Ex Taq (5U/µL) 0.25µL, total volume 50µL.

The PCR amplification was performed as follows: pre-denaturation at 94°C for 5 min, denaturation at 94°C for 30s, annealing at 52°C for 30s, extension at 72°C for 60s (30 cycles), and overextension at 72°C for 10 min.

The correctly sequenced pXMJ19-PtsS and pXMJ19-PtsS^{M162T} plasmids were electro-transformed into the patented strain YP97158 of L-lysine-producing bacteria, and the single colony produced by the culture were identified by PCR with primers M13/P18. The PCR amplified fragment containing a fragment of size 2362 bp was the positive strain, which were named as YPL-4-038 (without mutation point) and YPL-4-039 (with mutation point).

### (5) Construction of engineering strains that lacks the PtsS gene on its genome

According to the genome sequence of Corynebacterium glutamicum ATCC13032 published by NCBI, two pairs of primers for amplifying the fragments at both ends of the coding region of the PtsS gene were synthesized as upstream and downstream homology arm fragments. The primers were designed as follows (synthesized by Shanghai Yingjun company):

P19:5' CAGTGCCAAGCTTGCATGCCTGCAGGTCGACTCTAGGCCGTTAT C AAT
CAAGCGC3'(SEQ ID NO:83)

P20:5'CGCCAAAGTCTGGATGATGGTGGAAAGATTGTGGTGGC 3'(SEQ ID NO:84)
P21:5'GCCACCACAATCTTTCCACCATCATCCAGACTTTGGCGATCC 3'(SEQ ID NO:85)

P22:5' CAGCTATGACCATGATTACGAATTCGAGCTCGGTACCCGCAGTA AA
CGGTTCTGATGC3'(SEQ ID NO:86)

Construction method: Corynebacterium glutamicum ATCC13032 was used as a template, PCR amplification was carried out with primers P19/P20 and P21/P22, respectively, to obtain an upstream homology arm fragment of 794bp and a downstream homology arm fragment of 703bp, and then the entire homology arm fragment of 1459bp was obtained by OVERLAP PCR with primers P19/P22. The amplified product was electrophoresed and purified using a column DNA gel recovery kit, and the recovered DNA fragment was ligated with purified pK18mobsacB plasmid (purchased from Addgene Company, which was double digested with Xbal I/BamH I respectively) after double digestion with NEBuilder enzyme(purchased from NEB Company) at 50°C for 30 min, and the single clone grown after the transformation of the ligation product was identified by PCR with M13 primer to obtain the positive knockout vector pK18-ΔPtsS, which was sent for sequencing. This plasmid contained kanamycin resistance as a selectable marker.

PCR system: 10×Ex Taq Buffer 5µL, dNTP Mixture (2.5mM each) 4µL, Mg ²⁺ (25mM) 4µL, primers (10pM) 2µL each, Ex Taq (5U/µL) 0.25µL, total volume 50µL. The PCR amplification was performed as follows: pre-denaturation at 94°C for 5 min, denaturation at 94°C for 30s, annealing at 52°C for 30s, extension at 72°C for 90s (30 cycles), and overextension at 72°C for 10 min.

The correctly sequenced knockout plasmid pK18-ΔPtsS was electro-transformed into the patented strain YP97158 of producing lysine, and the single colony produced by the culture was identified by PCR with the following primers (synthesized by Shanghai Yingjun Company):
P23:5'TGTCAAAGATGCGTAGAGTCAC 3'(SEQ ID NO:87)
P24:5'GGTTTCATTCGCTTTCCG 3'(SEQ ID NO:88)

The strains with 758bp and 2249bp bands simultaneously amplified by the above PCR were positive strains, and the strain with 2249bp band only amplified was the original strains. Positive strains were screened on 15% sucrose medium and cultured on kanamycin-containing and kanamycin-free medium, respectively. The strains that grew on the medium without kanamycin but did not grow on the medium containing kanamycin were further identified by PCR using P23/P24 primers, and the amplified strains with a size of 758 bp were the positive strain in which the coding region of the PtsS gene had been knocked out. The positive strain PtsS fragment was amplified by PCR with P23/P24 primers again, and ligated to the PMD19-T vector for sequencing. The correctly sequenced strain was named YPL-4-040.

### (6) L-Lysine Fermentation Experiment

The strains constructed in the examples and the original strain YP97158 were subjected to fermentation experiments in the BLBIO-5GC-4-H fermentor (purchased from Shanghai Bailun Biotechnology Co., Ltd.) with the medium shown in Table 10 and the control process shown in Table 11. Each strain was repeated three times, and the results were shown in Table 12.

**Table 10 Formula of fermentation medium**

| Ingredient | Recipe |
|---|---|
| Starch hydrolyzed sugar | 30g/L |
| Ammonium sulfate | 12g/L |
| Magnesium sulfate | 0.87g/L |
| Molasses | 20g/L |
| Acidified corn steep liquor | 3mL/L |
| Phosphoric acid | 0.4mL/L |
| Potassium chloride | 0.53g/L |
| Defoamer (2% GPE) | 4mL/L |
| Ferrous sulfate | 120mg/L |
| Manganese sulfate | 120mg/L |
| Niacinamide | 42mg/L |
| Calcium pantothenate | 6.3mg/L |
| Vitamin B1 | 6.3mg/L |
| Copper and zinc salt solution | 0.6g/L |
| Biotin | 0.88mg/L |

**Table 11 Fermentation control process**

| | | | |
|---|---|---|---|
| Correction DO 100% | Temperature 37°C, air volume 4L/min, speed 1000rpm, tank pressure Ompa, calibrate after 5min | | |
| Inoculation amount | 10% | Culture temperature°C | 37°C |
| pH | pH6.9±0.05 | Dissolved oxygen DO | 10-30% |
| Initial conditions | Temperature 37°C, pH6.9, Tank pressure OMpa, Air volume 3L/min, Speed 550rpm | | |
| Whole process control | Whole process control 1. When the dissolved oxygen was less than 30%, increase the speed by 750rpm→800rpm→air volume 4L/min→850rpm→950rpm; 2, Fermentation 6h lift tank pressure 0.01Mpa; 12h lift tank pressure 0.02Mpa→0.03Mpa→0.04Mpa→0.05Mpa | | |
| Residual sugar control | 0.1-0.2% before F12h; after F12h, combined with DO, it was required to control residual sugar 0.1-0.05% | | |
| Ammonia nitrogen control | 0.1-0.15 before F12h; F12-F32h 0.15-0.25; 0.1-0.15 after F32h | | |
| Feeding material | 25% ammonia water, 70% concentrated sugar, 50% ammonium sulfate,10% GPE | | |
| Fermentation cycle | Around 48h | | |

**Table 12 L-lysine fermentation experiment results**

| Strain | L-lysine production (g/l00ml) | Transformation rate (%) |
|---|---|---|
| YP97158 | 18.9 | 65.2 |
| YPL-4-035 | 19.6 | 66.4 |
| YPL-4-036 | 19.4 | 65.8 |
| YPL-4-037 | 19.8 | 66.7 |
| YPL-4-038 | 19.5 | 66.2 |
| YPL-4-039 | 19.9 | 66.3 |
| YPL-4-040 | 18.0 | 63.8 |

The results were shown in Table 12. Point mutation PtsS^{M162T} and overexpression of the coding region of the PtsS gene in Corynebacterium glutamicum contributed to the improvement of L-lysine production and transformation rate, while weakening or knocking out the gene was not conducive to the accumulation of L-lysine, and at the same time reduced the transformation rate.

(7) The PtsS gene was introduced into the glutamic acid-producing strain and overexpressed, or the coding region of the PtsS gene was subjected to point mutation ptsS^{M162T} and overexpression, and fermentation experiments were carried out.

According to the method of this examples (1)-(5), the same primers and experimental conditions were used, and Corynebacterium ATCC13869 was used as the starting bacterium, and the bacterium of ATCC 13869 was used as the expression bacterium to obtain a glutamic acid-producing engineering strain of ptsS^{M162T} containing the point mutation, a glutamic acid-producing engineering strain overexpressing the ptsS and ptsS^{M162T} genes on the genome, and a glutamic acid-producing engineering strain overexpressing ptsS and ptsS^{M162T} genes on plasmids, and a glutamic acid-producing engineering strain lacking the ptsS gene on the genome.

The strains constructed in the examples and the original strain ATCC 13869 were subjected to fermentation experiments in the BLBIO-5GC-4-H fermentor (purchased from Shanghai Bailun Biotechnology Co., Ltd.) with the medium shown in Table 13 and the control process shown in Table 14. Each strain was repeated three times, and the results were shown in Table 15.

**Table 13 Formula of fermentation medium**

| Reagent name | Proportion |
|---|---|
| Glucose | 5.0g/L |
| Phosphoric acid | 0.38g/L |
| Magnesium sulfate | 1.85g/L |
| Potassium chloride | 1.6g/L |
| Biotin | 550ug/L |
| Vitamin B1 | 300ug/L |
| Ferrous sulfate | 10mg/L |
| Manganese sulfate | 10g/dl |
| KH₂PO₄ | 2.8g/L |
| Vitamin C | 0.75mg/L |
| Vitamin B12 | 2.5ug/L |
| para aminobenzoic acid | 0.75mg/L |
| Defoamer | 0.0015ml/dl |
| Betaine | 1.5g/L |
| cane molasses | 7ml/L |
| corn syrup | 77ml/L |
| aspartic acid | 1.7g/L |
| hair powder | 2g/L |

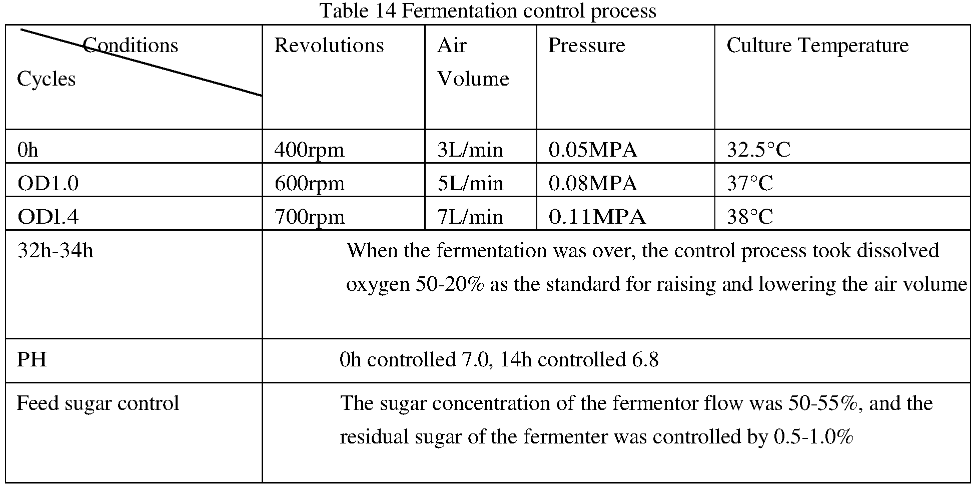

**Table 15 L-glutamic acid fermentation experiment results**

| Strain | L-glutamic acid production (g/l) | Transformation rate(%) |
|---|---|---|
| ATCC13869 | 101.0 | 45.8 |
| YPG-007 | 107.5 | 46.4 |
| YPG-008 | 107.9 | 46.8 |
| YPG-009 | 107.2 | 46.3 |
| YPG-010 | 107.6 | 46.7 |
| YPG-011 | 107.8 | 46.2 |
| YPG-012 | 96.5 | 45.2 |

The results were shown in Table 15. Point mutation PtsS^{M162T} and/or overexpression of the coding region of the PtsS gene in Corynebacterium glutamicum contributed to the improvement of L-lysine production and transformation rate, while weakening or knocking out the gene was not conducive to the accumulation of L-lysine, and at the same time reduced the transformation rate.

The embodiments of the present invention have been described above. However, the present invention is not limited to the above-described embodiments. Any modification, equivalent replacement, improvement, etc. within the spirit and principle of the present invention should be included within the protection scope of the present invention.

## Claims

1. A microorganism or bacterium that generates an L-amino acid, **characterized in that** the microorganism or bacterium has an improved expression of the polynucleotide coding the amino acid sequence of SEQ ID NO:3, and/or an improved expression of the polynucleotide coding the amino acid sequence of SEQ ID NO:35, and/or an improved expression of the polynucleotide coding the amino acid sequence of SEQ ID NO: 63;
preferably, the improved expression is an enhanced expression of the polynucleotide coding the amino acid sequence, or the polynucleotide coding the amino acid sequence has a point mutation, or the polynucleotide coding the amino acid sequence has a point mutation and the expression is enhanced.

2. The microorganism or bacterium according to claim 1, **characterized in that** the point mutation of the polynucleotide coding the amino acid sequence of SEQ ID NO: 3 makes the glutamic acid at position 170 of the amino acid sequence of SEQ ID NO: 3 replaced by different amino acids; preferably the glutamic acid at position 170 is replaced by lysine;
the point mutation of the polynucleotide coding the amino acid sequence of SEQ ID NO: 35 makes the leucine at position 31 of the amino acid sequence of SEQ ID NO: 35 replaced by different amino acids; preferably leucine at position 31 is replaced by arginine;
the point mutation of the polynucleotide coding the amino acid sequence of SEQ ID NO:63 makes the methionine at position 162 of the amino acid sequence of SEQ ID NO:63 replaced by different amino acids; preferably, the methionine at position 162 is replaced by threonine.

3. The microorganism or bacterium according to any one of claims 1-2, **characterized in that** the polynucleotide coding the amino acid sequence of SEQ ID NO:3 comprises the nucleotide sequence of SEQ ID NO:1; the polynucleotide coding the amino acid sequence of SEQ ID NO: 35 comprises the nucleotide sequence of SEQ ID NO:33; the polynucleotide coding the amino acid sequence of SEQ ID NO:63 comprises the nucleotide sequence of SEQ ID NO:61.

4. The microorganism or bacterium according to any one of claims 1-3, **characterized in that** the polynucleotide sequence with point mutation is formed by the mutation of the 508^{th} base of the polynucleotide sequence shown in SEQ ID NO: 1; preferably, the mutation comprises the mutation of the 508^{th} base of the polynucleotide sequence shown in SEQ ID NO: 1 from guanine (G) to adenine (A); preferably, the polynucleotide sequence with point mutation comprises the polynucleotide sequence shown in SEQ ID NO: 2;
the polynucleotide with point mutation is formed by the mutation of the 92^{nd} base of the polynucleotide sequence shown in SEQ ID NO:33; preferably, the mutation comprises the mutation of the 92^{nd} base of the polynucleotide sequence shown in SEQ ID NO:33 from thymine (T) to guanine (G); preferably, the polynucleotide sequence with point mutation comprises the polynucleotide sequence shown in SEQ ID NO: 34;
the polynucleotide with point mutation is formed by the mutation of the 485^{th} base of the polynucleotide sequence shown in SEQ ID NO:61; preferably, the mutation comprises the mutation of the 485^{th} base of the polynucleotide sequence shown in SEQ ID NO:61 from thymine (T) to cytosine (C); preferably, the polynucleotide sequence with point mutation comprises the polynucleotide sequence shown in SEQ ID NO:62.

5. The microorganism or bacterium according to any one of claims 1-4, **characterized in that** the microorganism is Corynebacterium glutamicum; preferably YP97158 or ATCC 13869.

6. A polynucleotide sequence, comprising the polynucleotide coding the amino acid sequence shown in SEQ ID NO: 3, wherein the 170^{th} glutamic acid is replaced by different amino acids; preferably the 170^{th} glutamic acid is replaced by lysine; preferably the polynucleotide sequence comprises a polynucleotide coding the amino acid sequence shown in SEQ ID NO:4; preferably the polynucleotide sequence is formed by the mutation of the 508^{th} base of the polynucleotide sequence shown in SEQ ID NO: 1; preferably the mutation is the mutation of the 508^{th} base of the polynucleotide sequence shown in SEQ ID NO: 1 from guanine (G) to adenine (A); preferably the polynucleotide sequence comprises the polynucleotide sequence shown in SEQ ID NO: 2;
and/or,
comprising the polynucleotide coding the amino acid sequence shown in SEQ ID NO: 35, wherein the 31^{st} leucine is replaced by different amino acids; preferably the 31^{st} leucine is replaced by arginine; preferably, the polynucleotide sequence comprises a polynucleotide coding the amino acid sequence shown in SEQ ID NO: 36; preferably the polynucleotide sequence is formed by the mutation of the 92^{nd} base of the polynucleotide sequence shown in SEQ ID NO: 33; preferably the mutation is the mutation of the 92^{nd} base of the polynucleotide sequence shown in SEQ ID NO: 33 from thymine (T) to guanine (G); preferably the polynucleotide sequence comprises the polynucleotide sequence shown in ID NO: 34;
and/or,
comprising the polynucleotide coding the amino acid sequence shown in SEQ ID NO: 63, wherein the 162^{nd} methionine is replaced by different amino acids; preferably the 162^{nd} methionine is replaced by threonine; preferably the polynucleotide sequence comprises the polynucleotide sequence coding the amino acid shown in SEQ ID NO: 64; preferably the polynucleotide sequence is formed by the mutation of the 485^{th} base of the polynucleotide sequence shown in SEQ ID NO: 61; preferably the mutation comprises the mutation of 485^{th} base of the polynucleotide sequence shown in SEQ ID NO: 61 from thymine (T) to cytosine (C); preferably, the polynucleotide sequence comprises the polynucleotide sequence shown in SEQ ID NO:62.

7. An amino acid sequence shown in SEQ ID NO:4 or SEQ ID NO:36 or SEQ ID NO:64.

8. A recombinant vector, comprising the polynucleotide sequence according to claim 6.

9. A recombinant strain, comprising the polynucleotide sequence according to claim 6.

10. A method for producing an L-amino acid, comprising: culturing the microorganism or bacterium of any one of claims 1-5 or the recombinant strain of claim 9, and recovering L-amino acids from the culture; wherein preferably the L-amino acid is L-glutamic acid or L-lysine.
